(19) **Europäisches Patentamt / European Patent Office / Office européen des brevets**

(11) **EP 4 057 297 B1**

(12) **EUROPEAN PATENT SPECIFICATION**

(45) Date of publication and mention of the grant of the patent:
**05.11.2025 Bulletin 2025/45**

(51) International Patent Classification (IPC):
*G16H 50/50* (2018.01)    *G06N 3/045* (2023.01)
*G06N 3/047* (2023.01)    *G06N 3/048* (2023.01)
*G06N 7/01* (2023.01)    *G16H 50/20* (2018.01)
*G06N 3/08* (2023.01)

(21) Application number: **21305306.9**

(22) Date of filing: **12.03.2021**

(52) Cooperative Patent Classification (CPC):
**G16H 50/20; G06N 3/045; G06N 3/047;
G06N 3/048; G06N 3/0499; G06N 3/08; G06N 3/09;
G06N 3/0985; G06N 7/01; G16H 50/50**

(54) **MACHINE-LEARNING WITH RESPECT TO MULTI-STATE MODEL OF AN ILLNESS**

MASCHINENLERNEN BEZÜGLICH EINES MEHRFACHZUSTANDSMODELLS EINER KRANKHEIT

APPRENTISSAGE AUTOMATIQUE EN CE QUI CONCERNE LE MODÈLE MULTI-ÉTATS D'UNE MALADIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.09.2022 Bulletin 2022/37**

(73) Proprietor: **Dassault Systèmes**
**78140 Vélizy-Villacoublay (FR)**

(72) Inventors:
• **COTTIN, Aziliz**
**78946 Vélizy-Villacoublay Cedex (FR)**
• **PECUCHET, Nicolas**
**78946 Vélizy-Villacoublay Cedex (FR)**
• **GUILLOUX, Agathe**
**91037 Evry Cedex (FR)**
• **KATSAHIAN, Sandrine**
**75006 PARIS (FR)**

(74) Representative: **Bandpay & Greuter**
**11 rue Christophe Colomb**
**75008 Paris (FR)**

(56) References cited:
• **STEFAN GROHA ET AL: "A General Framework for Survival Analysis and Multi-State Modelling", ARXIV.ORG, CORNELL UNIVERSITY LIBRARY, 201 OLIN LIBRARY CORNELL UNIVERSITY ITHACA, NY 14853, 15 February 2021 (2021-02-15), XP081878777**
• **BENNIS ACHRAF ET AL: "Estimation of Conditional Mixture Weibull Distribution with Right Censored Data Using Neural Network for Time-to-Event Analysis", 6 May 2020, ADVANCES IN INTELLIGENT DATA ANALYSIS XIX; [LECTURE NOTES IN COMPUTER SCIENCE; LECT.NOTES COMPUTER], SPRINGER INTERNATIONAL PUBLISHING, CHAM, PAGE(S) 687 - 698, ISBN: 978-3-540-71008-0, ISSN: 0302-9743, XP047551038**
• **PING WANG ET AL: "Machine Learning for Survival Analysis: A Survey", ARXIV.ORG, CORNELL UNIVERSITY LIBRARY, 201 OLIN LIBRARY CORNELL UNIVERSITY ITHACA, NY 14853, 15 August 2017 (2017-08-15), XP080953051**

**Description**

**TECHNICAL FIELD**

The disclosure relates to the field of biostatistics, and more specifically to methods, computer programs and devices related to machine-learning a function configured, based on input covariates representing medical characteristics of a patient, with respect to a multi-state model of an illness having states and transitions between the states, to output a distribution of transition-specific probabilities.

**BACKGROUND**

Disease prognosis is of major importance for physicians when making medical decisions, and it involves specialized algorithms to estimate the risks of a patient. Event history analysis, also known as survival analysis, aims at predicting the time until the occurrence of one or more future events of interest, and it is used in multiple areas including healthcare, in the context of disease prognosis. In particular, survival analysis is very often used in healthcare to model patient outcome survival in order to assess treatment efficacy. In clinical practice, clinicians may be more interested in the complete evolution of a disease and not only in a unique or composite event. The multi-state approach has thus been developed as a generalization of survival analysis where multiple events can occur successively over time (see Reference 1).

The illness-death model is a particular multi-state model composed of three states: "healthy", "relapsed" or "diseased", and "dead". This is the most frequent structure used to follow the evolution of cancer patients through an intermediate non-fatal relapse state and a death state, such as in ovarian cancer (see Reference 2) or in chronic myeloid leukemia (see Reference 3). Other applications of the illness-death model include the Alzheimer's disease (see Reference 4) and cardiovascular diseases (see Reference 5).

There are two main literature streams on event history analysis in this context.

The first stream is based on traditional statistic theories, including three approaches. (i) The non-parametric approaches including in particular the Kaplan Meier estimator (see Reference 6) and the Nelson-Aalen estimator (see Reference 7). They are traditionally used to model the risk of an event without making any assumption on the distribution of the event times, and they do not enable to make individualized modeling. (ii) Parametric approaches enable individualized modeling. The event times are related to individual covariates through a linear regression function, and they are distributed according to an underlying probability distribution. (iii) Semi-parametric approaches allow a compromise between non-parametric and parametric models. They introduce covariate effects through a linear regression function, but they do not make any assumptions regarding the distribution of the event times. The Cox proportional hazard (P.H.) model is the most widely used semi-parametric model in multivariate survival analysis (see Reference 8). In multi-state analysis, most of the existing literature describes the risk of transiting between states using transition-specific Cox P.H. models as (semi-) Markov processes (see References 9 and 10). However, these traditional methods rely on strong statistical assumptions about either the distribution of the event times, or about the relation between the covariates and the event times. In particular, the Cox P.H. model makes a linear assumption about the relationship between the covariates and the risk of event. This assumption shows limitations in many real-world applications, as the effect of a covariate may vary in a non-linear way in response to variation of the risk of event. The interactions between covariates are not considered by default in the Cox model. This limits the application of this model to big data, as most variables are not directly related to the modeled outcome but will interact with the covariates effect. For instance, a genetic variation in a metabolic pathway might not directly impact the risk of cancer relapse, but it might reduce or enhance the anti-tumor treatment effect. These limitations of the Cox model are widely known in a clinical setting.

To address these challenges, a second variety of literature has arisen employing new machine learning algorithms. In particular, neural networks have been developed to extend the Cox P.H. model in a statistical assumption-free framework. Traditional artificial neural networks have in particular been successfully introduced for survival analysis by Faraggi and Simon (see Reference 11). More recently, deep neural networks have been extended by Luck et al. (see Reference 12), Katzman et al. (see Reference 13), Fotso (see Reference 14), Kvamme et al. (see Reference 15), among others (see also References 16, 17 and 18). By employing best state-of-the-art deep learning methods and larger clinical datasets, they show significant improvements in predicting patients' survival as compared to the Cox P.H. model. Regardless, their approaches are still limited to the case of a unique clinical event. In addition, most of the recent methods directly predict a discrete-time distribution of the event times as an output of the neural network. As an approximation for continuous-time survival data, they all perform a division of the continuous time scale into discrete-time intervals. This leads to a relatively significant approximation error.

Within this context, there is thus a need for an improved solution for determining accurate patient data with respect to a multi-state model of an illness, for example so as to provide accurate individual predictions of the evolution of an illness with respect to an illness-death multi-state model.

**CITED REFERENCES**

1. Webster AJ. Multi-stage models for the failure of complex systems, cascading disasters, and the onset of disease. PloS one 2019; 14(5): e0216422.
2. Eulenburg C, Mahner S, Woelber L, Wegscheider K. A systematic model specification procedure for an illness-death model without recovery. PloS one 2015; 10(4): e0123489.
3. Iacobelli S, Carstensen B. Multiple time scales in multi-state models. Statistics in medicine 2013; 32(30): 5315-5327.
4. Commenges D, Joly P, Letenneur L, Dartigues JF. Incidence and mortality of Alzheimer's disease or dementia using an illness-death model. Statistics in medicine 2004; 23(2): 199-210.
5. Ramezankhani A, Blaha MJ, Mirbolouk hM, Azizi F, Hadaegh F. Multi-state analysis of hypertension and mortality: application of semi-Markov model in a longitudinal cohort study. BMC Cardiovascular Disorders 2020; 20(1): 1-13.
6. Kaplan EL, Meier P. Nonparametric estimation from incomplete observations. Journal of the American statistical association 1958; 53(282): 457-481.
7. Aalen O. Nonparametric inference for a family of counting processes. The Annals of Statistics 1978: 701-726.
8. Cox DR. Regression models and life-tables. Journal of the Royal Statistical Society: Series B (Methodological) 1972; 34(2): 187-202.
9. De Wreede LC, Fiocco M, Putter H. The mstate package for estimation and prediction in non-and semi-parametric multistate and competing risks models. Computer methods and programs in biomedicine 2010; 99(3): 261-274.
10. Andersen PK, Borgan O, Gill RD, Keiding N. Statistical models based on counting processes. Springer Science & Business Media . 2012.
11. Faraggi D, Simon R. A neural network model for survival data. Statistics in medicine 1995; 14(1): 73-82.
12. Luck M, Sylvain T, Cardinal H, Lodi A, Bengio Y. Deep learning for patient-specific kidney graft survival analysis. arXiv preprint arXiv:1705.10245 2017.
13. Katzman JL, Shaham U, Cloninger A, Bates J, Jiang T, Kluger Y. DeepSurv: personalized treatment recommender system using a Cox proportional hazards deep neural network. BMC medical research methodology 2018; 18(1): 24.
14. Fotso S. Deep neural networks for survival analysis based on a multi-task framework. arXiv preprint arXiv:1801.05512 2018.
15. Kvamme H, Borgan Ø, Scheel I. Time-to-event prediction with neural networks and Cox regression. Journal of Machine Learning Research 2019; 20(129): 1-30.
16. Giunchiglia E, Nemchenko A, Schaar v. dM. Rnn-surv: A deep recurrent model for survival analysis. In: Springer. ; 2018: 23-32.
17. Ren, K., Qin, J., Zheng, L., Yang, Z., Zhang, W., Qiu, L., & Yu, Y. (2019). Deep Recurrent Survival Analysis. Proceedings of the AAAI Conference on Artificial Intelligence, 33(01), 4798-4805.
18. Gensheimer MF, Narasimhan B. A scalable discrete-time survival model for neural networks. PeerJ2019; 7: e6257.
19. Lee C, Zame WR, Yoon J, Schaar v. dM. Deephit: A deep learning approach to survival analysis with competing risks. In: ; 2018.
20. Kalbfleisch JD, Prentice RL. The statistical analysis of failure time data. 360. John Wiley & Sons. 2011.
21. Friedman M, others . Piecewise exponential models for survival data with covariates. The Annals of Statistics 1982; 10(1): 101-113.
22. Kvamme H, Borgan Ø. Continuous and discrete-time survival prediction with neural networks. arXiv preprint arXiv:1910.06724 2019.
23. Andersen PK, Borgan Ø. Counting Process Models for Life History Data: A Review. Preprint series. Statistical Research Report http://urn. nb. no/URN: NBN: no-23420 1984.
24. Meira-Machado L, Uña-Álvarez dJ, Cadarso-Suárez C, Andersen PK. Multi-state models for the analysis of time-to-event data. Statistical methods in medical research 2009; 18(2): 195-222.
25. Meira-Machado L, Sestelo M. Estimation in the progressive illness-death model: A nonexhaustive review. Biometrical Journal 2019; 61(2): 245-263.
26. Triebel H. Theory of Function Spaces, vol. 78 of. Monographs in mathematics 1983.
27. Putter H, Fiocco M, Geskus RB. Tutorial in biostatistics: competing risks and multi-state models. Statistics in medicine 2007; 26(11): 2389-2430.
28. Ruder S. An overview of multi-task learning in deep neural networks. arXiv preprint arXiv:1706.05098 2017.
29. Lee C, Yoon J, Van Der Schaar M. Dynamic-deephit: A deep learning approach for dynamic survival analysis with competing risks based on longitudinal data. IEEE Transactions on Biomedical Engineering 2019; 67(1): 122-133.
30. Möst S. Regularization in discrete survival models. PhD thesis. lmu, 2014.

31. Tibshirani R, Saunders M, Rosset S, Zhu J, Knight K. Sparsity and smoothness via the fused lasso. Journal of the Royal Statistical Society: Series B (Statistical Methodology) 2005; 67(1): 91-108.

32. AalenOO, JohansenS. An empirical transition matrix for non-homogeneous Markov chains based on censored observations. Scandinavian Journal of Statistics 1978: 141-150.

33. Jacqmin-Gadda H, Blanche P, Chary E, Touraine C, Dartigues JF. Receiver operatingcharacteristic curveestimation for time to event with semicompeting risks and interval censoring. Statistical methods in medical research 2016; 25(6): 2750-2766.

34. Spitoni C, Lammens V, Putter H. Prediction errors for state occupation and transition probabilities in multi-state models. Biometrical Journal 2018; 60(1): 34-48.

35. Jackson CH. flexsurv: a platform for parametric survival modeling in R. Journal of statistical software 2016; 70.

36. Royston P, Altman DG. External validation of a Cox prognostic model: principles and methods. BMC medical research methodology 2013; 13(1): 33.

37. Bergstra J, Bengio Y. Random search for hyper-parameter optimization. The Journal of Machine Learning Research 2012; 13(1): 281-305.

38. Wilcoxon F. Individual comparisons by ranking methods. In: Springer. 1992 (pp. 196-202).

39. Bender R, Augustin T, Blettner M. Generating survival times to simulate Cox proportional hazards models. Statistics in medicine 2005; 24(11): 1713-1723.

40. Van Wieringen WN, Kun D, Hampel R, Boulesteix AL. Survival prediction using gene expression data: a review and comparison. Computational statistics & data analysis 2009; 53(5): 1590-1603.

41. Yousefi S, Amrollahi F, Amgad M, et al. Predicting clinical outcomes from large scale cancer genomic profiles with deep survival models. Scientific reports 2017; 7(1): 1-11.

42. Ching T, Zhu X, Garmire LX. Cox-nnet: an artificial neural network method for prognosis prediction of high-throughput omics data. PLoS computational biology 2018; 14(4): e1006076.

43. Kuleshov MV, Jones MR, Rouillard AD, et al. Enrichr: a comprehensive gene set enrichment analysis web server 2016 update. Nucleic acids research 2016; 44(W1): W90-W97.

44. Liberzon A, Birger C, Thorvaldsdóttir H, Ghandi M, Mesirov JP, Tamayo P. The molecular signatures database hallmark gene set collection. Cell systems 2015; 1(6): 417-425.

45. Wu Y, Sarkissyan M, Vadgama JV. Epithelial-mesenchymal transition and breast cancer. Journal of clinical medicine 2016; 5(2): 13.

46. Lee DS, Ezekowitz JA. Risk stratification in acute heart failure. Canadian Journal of Cardiology 2014; 30(3): 312-319.

47. Park S, Han W, Kim J, et al. Risk factors associated with distant metastasis and survival outcomes in breast cancer patients with locoregional recurrence. Journal of breast cancer 2015; 18(2): 160.

48. Lip GY, Nieuwlaat R, Pisters R, Lane DA, Crijns HJ. Refining clinical risk stratification for predicting stroke and thromboembolism in atrial fibrillation using a novel risk factor-based approach: the euro heart survey on atrial fibrillation. Chest 2010; 137(2): 263-272.

49. Sparano JA, Crager MR, Tang G, Gray RJ, Stemmer SM, Shak S. Development and validation of a tool integrating the 21gene recurrence score and clinical-pathological features to individualize prognosis and prediction of chemotherapy benefit in early breast cancer. Journal of Clinical Oncology 2020: JCO-20.

50. Ancona M, Ceolini E, Öztireli C, Gross M. Towards better understanding of gradient-based attribution methods for deep neural networks. arXiv preprint arXiv:1711.06104 2017.

51. Jackson C. Multi-state modeling with R: the msm package. Cambridge, UK 2007: 1-53.

A neural network approach designed to explicitly handle multi-state survival models without using common simplifying assumptions and to continuously model both time and probability distributions is described in Groha, S., Schmon, S.M. and Gusev, A., 2020. A general framework for survival analysis and multi-state modelling. arXiv preprint arXiv:2006.04893.

## SUMMARY

It is therefore provided a computer-implemented method for machine-learning a function as defined in appended independent claim 1. The function is configured, based on input covariates representing medical characteristics of a patient, with respect to a multi-state model of an illness having states and transitions between the states, to output a distribution of transition-specific probabilities for each interval of a set of intervals. The set of intervals forms a subdivision of a follow-up period. The machine-learning method comprises providing an input dataset of covariates and time-to-event data of a set of patients. The machine-learning method also comprises training the function based on the input dataset. The machine-learning method comprises the following:

- the function comprises a covariate-shared subnetwork and a transition-specific subnetwork per transition;
- the covariate-shared subnetwork comprises a respective fully connected neural network and each transition-specific subnetwork comprises a fully connected neural network;
- the covariate-shared subnetwork comprises a respective non-linear activation function and each transition-specific subnetwork comprises a respective non-linear activation function;
- each transition-specific subnetwork is followed by a softmax layer;
- the multi-state model comprises competing transitions, the transition-specific subnetworks of the competing transitions share a common softmax layer;
- the training comprises minimizing a loss function which includes a likelihood term, and a regularization term that penalizes, in weight matrices, first order differences of weights associated with two adjacent time intervals, and/or, in bias vectors, first order differences of biases associated with two adjacent time intervals;
- the multi-state model is an illness-death model;
- the illness is a cancer disease, for example a breast cancer, or a disease characterized by an intermediate state and a final state;
- the illness is characterized by states representing a condition of the patient with respect to the illness: clinical symptoms (e.g., hemorrhage, dyspnea at rest), or radiological findings (e.g., osteopenia, bone fracture), or biological measures (e.g., creatinine clearance); and/or
- the medical characteristics comprise characteristics representing a general state of the patient and/or characteristics representing a condition of the patient with respect to the illness.

It is further provided a method of use of a function machine-learnt according to the above machine-learning method, as defined in appended claim 3. The use method comprises providing input covariates representing medical characteristics of a patient, and applying the function to the input covariates. Thereby, the use method outputs, with respect to a multi-state model of an illness having states and transitions between the states, a distribution of transition-specific probabilities for each interval of a set of time intervals. The set of time intervals forms a subdivision of a follow-up period.

The use method may further comprise one or more of the following:

- computing one or more transition-specific cumulative incidence functions, and optionally displaying said one or more transition-specific cumulative incidence functions;
- identifying relapse risk and/or death risk associate to the patient; and/or
- determining a treatment, a treatment adaptation, a follow-up visit, and/or a surveillance with diagnostic tests, for example based on the identified relapse risk and/or death risk.

It is further provided a computer program including instructions for performing the above machine-learning method, as defined in appended claim 5.

It is further provided a computer program including instructions for performing the above use method, as also defined in appended claim 5.

It is further provided a device comprising a data storage medium having recorded thereon any or both the above computer programs, as defined in appended claim 6. The device may form or serve as a non-transitory computer-readable medium, for example on a SaaS (Software as a service) or other server, or a cloud based platform, or the like. The device may alternatively comprise a processor coupled to the data storage medium. The device may thus form a computer system in whole or in part (e.g., the device is a subsystem of the overall system). The system may further comprise a graphical user interface coupled to the processor.

## BRIEF DESCRIPTION OF THE DRAWINGS

Non-limiting examples will now be described in reference to the accompanying drawings, where:

- FIG. 1 shows an illustration of an illness-death model;
- FIG. 2 shows a discretization of a time axis into $K$ time intervals;
- FIG. 3 shows the architecture of an example neural network function;
- FIG.s 4 to 6 show obtained results; and
- FIG. 7 shows an example of the system.

## DETAILED DESCRIPTION

A computer-implemented method is provided for machine-learning a function (i.e., determining via a machine-learning process a neural network function, that is, a function comprising or consisting of one or more neural networks).

The function is configured to be fed with an input and to provide a certain output, in accordance with the following. The input is a plurality of covariates (i.e., pieces of data) that represent medical characteristics of a patient. The output is defined with respect to (i.e., in reference to) a multi-state model of an illness (e.g., an illness-death model). In other words, the multi-state model is predetermined. The (e.g., illness-death) multi-state model has (i.e., is characterized by) (e.g., three) states and (e.g., irreversible) transitions between the states. Given this, the output is a distribution of transition-specific (i.e., transition-specific) probabilities for each interval of a set of intervals. The set of intervals is such that it forms a subdivision of a follow-up period. In other words, the output is a distribution of probabilities constant values, with exactly one probability constant value per each respective time interval of a follow-up period and per each respective transition of the multi-state model. Said probability constant value (e.g., a single number, e.g., between 0 and 1) represents probability that the patient experiences the respective transition (and thus corresponding changes states) during said respective time interval. The machine-learning method comprises providing an input dataset of covariates and (e.g., illness-death) time-to-event data of a set of patients. The machine-learning method further comprises training the function based on the input dataset.

This forms an improved solution for determining accurate patient data with respect to a multi-state model of an illness, and in examples for providing accurate individual predictions of the evolution of an illness with respect to an illness-death multi-state model.

In particular, after such offline learning/training, the machine-learnt function may be used online/inline by providing input covariates of a patient, i.e., a plurality of covariates (i.e., pieces of data) that represent medical characteristics of said patient. The patient may be a new patient, i.e., not part of those involved in the dataset used in the machine-learning method. The input covariates of this patient may be the same than those used during the training process. The use method may then comprise applying the function to the input covariates. This allows outputting, with respect to the (e.g., illness-death) multi-state model, a distribution of transition-specific probabilities for each interval of a set of time intervals forming a subdivision of a follow-up period.

Such distribution of transition-specific probabilities forms medical/healthcare data which can be used in any manner in the context of patient's follow-up, in particular for prognosis and/or treatment decision-making. The methods thus allow notably to perform disease prognosis within an event history analysis framework. The use method in particular forms an algorithm for individual prognostication in a multi-state illness model (such as a three-state illness-death model, as illustrated later). By applying a multi-state analysis, the methods achieve high accuracy, as true transitions between real states of the illness are modeled.

By application of a neural network approach to multi-state analysis (for example for an illness-death process), the methods can perform a non-linear processing of the covariates. The function (i.e., neural network) can indeed fit non-linear patterns in the data, e.g., by using multiple (e.g., fully-connected) layers and/or one or more non-linear activation functions. This addresses the linear limitation of the Cox P.H. model within the (e.g., illness-death) modeling framework.

The outputted distribution of transition-specific probabilities amounts to densities of probabilities of occurrence of each transition over the follow-up period. Thus, the methods offer a well-approximated continuous-time framework (instead of a discrete-time framework, prone to higher approximation errors). In other words, the use method achieves high accuracy. Yet, the methods assume that the transition-specific density probabilities are piecewise constant, as one probability (of constant value) is determined per interval. This limits approximation error, e.g., compared to prior art discrete-time methods.

Section 1 below provides a general presentation of the methods and optional features thereof. Sections 2-4 present a specific implementation. Sections 5-8 discuss performance of the specific implementation. Section 9 discusses the system.

## 1 | GENERAL PRESENTATION

The multi-state model of the methods represents a stochastic process, and it may be any illness model having at least two states and at least two transitions, optionally at least three states and at least three transitions, wherein the patient is in exactly and only one state at a time.

The multi-state model may for example be an illness-death model.

The illness-death model comprises an initial (e.g., "Event-free") state representing non-illness of a patient, an intermediate (e.g., "Relapse" or "Disease") state representing illness of the patient, and an absorbing state (e.g., "Death") state representing death of the patient. The intermediate state may be referred to as "Relapse", but this is only a convention. Depending on the patient, an entry in the intermediate state may indeed either represent an actual relapse (for example if the considered disease is a cancer), or a first affection by the illness (for example if the considered disease is an infectious illness). Similarly, the state which represents non-illness of a patient is called "initial" state, but this is only a convention. In examples however, all the patients in the dataset may start in the initial state. Similarly, the use method may be applied for a patient in the initial state. In alternative examples, the dataset may comprise patients starting in the intermediate state according to their time-to-event data. Similarly in such a case, the use method may optionally be applied for a patient affected by the illness, thus having a state (at the time when the use method is performed) different from the "initial" state.

In examples, the patient in the use method and/or most (e.g., all or substantially all) patients of the set of patients (involved in the dataset) may initially be affected by the illness (i.e., patients presenting symptoms) or have previously been affected by the illness (i.e., patients presenting no symptom, that is, in remission). For instance, the patient in the use method and/or most (e.g., all or substantially all) patients of the set of patients (involved in the dataset) may have previously been affected by the illness but not affected anymore, thus in the initial state (i.e., patients presenting no symptom, that is, in remission). In other words, the initial state is a remission state and all patients considered in the methods are in the remission state. Thus, an entry in the intermediate state may represent actual relapse. The use method may thus serve to determine healthcare data for patients already followed for the illness. In other examples, the patient in the use method and/or most (e.g., all or substantially all) patients of the set of patients may initially be non-affected by the illness and have never been affected by the illness. Thus, first entry in the intermediate state may represent a first affection by the illness. The use method may thus serve to determine healthcare data for patients not followed for the illness and who have never been followed for that. In yet other examples, the set of patients may comprise a significant number of both types of patients and/or of patients in both states, and the use method may be indifferently applied to both types of patients and/or to a patient in any of both states. In such a case, the machine-learning can be seen as generalist.

In particular examples, the methods rely on a specific multi-state model which is the illness-death model, and a particular illness-death model which does not allow reversion to the initial state. The methods may apply the illness-death model to patients with a lethal disease (such as cancer) who are in remission. The methods may enable monitoring occurrence of relapse or death for these patients. Thus, all patients may start in the initial state in these particular examples.

In other particular examples, for instance applied to the case of infectious diseases, the multi-state model may alternatively comprise a possible return to the initial state. The multi-state model may be an infectious disease model.

The states of the illness-death model may optionally consist of exactly and only those three states. Alternatively, the states may comprise one or more other states, such as at least one additional intermediate state. For example, in the case of a multiple myeloma, the states may be the same except that there are two intermediate states: "biological relapse" and "clinical relapse".

The death-illness model comprises two competitive transitions, a first transition which is from the initial state to the intermediate state and a second transition which is from the initial state to the absorbing state. The death-illness model further comprises a third transition, which is from the intermediate state to the absorbing state. The transitions to the absorbing state are irreversible. The transitions from the intermediate state to the absorbing state are successive. The transition from the initial state to the intermediate state may be irreversible (i.e., the model comprises no other transition), or alternatively the illness-death model may further comprise a fourth transition, which is from the intermediate state to the initial state. In such a case, the transitions from the intermediate state are competing.

The illness may be any disease which can be characterized through an intermediate state and an absorbing state. In examples where the multi-state model is an illness-death model, the illness may be any deadly/lethal disease, i.e., a disease known for potentially incurring death at a statistical rate which is non-negligible over the human population (for example higher than 1% or 5%). The illness may for example be a cancer disease, for example a breast cancer. In other examples, the illness may be the Alzheimer's disease or a cardiovascular disease. Alternatively, the absorbing state may be non-lethal. For instance, age-related macular degeneration disease may evolve towards blindness which is the final absorbing state.

The covariates are respective pieces of data that form the argument of the machine-learnt function. Each such piece of data represents patient-wise a respective medical characteristic of the patient, which is relevant to the illness. Each piece of data may be in the form of a single value, a vector with a plurality of values/coordinates, or any other type of data structure conveying information on the patient, such as an image (e.g., a scan or radio image) or a list of biological analysis results. The neural network approach of the methods allows a high flexibility for the input covariates, in terms of number and heterogeneity of data.

Optionally, the input covariates may be provided to the function in the form of a unique vector, or alternatively the function may process the input covariates so as to form such a vector. For example, the function may concatenate values and/or vectors in a single vector. In the case of an image covariate, the function may comprise a respective convolutional neural network that converts the image in a vector, then concatenable to other values and/or vectors. The function may optionally be trained to handle missing data, i.e., null values. Additionally or alternatively, missing data may be completed upstream.

The medical characteristics may comprise characteristics representing a general state of the patient (i.e., so-called "core characteristics") and/or characteristics representing a condition of the patient with respect to the illness (i.e., "condition characteristics").

The core characteristics of a patient for example comprise their age, body mass index (BMI), comorbidities, sex, and/or any other general characteristic medically relevant. The condition characteristics may be more specific to the illness under study. For example, in the case of a breast cancer, the condition characteristics of a patient may comprise any one or any combination of inference on the menopausal status, Nottingham Prognostic Index (NPI), immunohistochemical oestrogen-receptor (ER) status, number of positive lymph nodes, cancer grade, tumor size, tumor histological type, cellularity, Her2 copy number, Her2 expression, ER Expression, progesterone (PR) expression, type of breast surgery, cancer molecular subtype (pam50 subgroup, integrative cluster), chemotherapy regime, hormone regime, and/or radiotherapy regime.

The number of covariates may be higher than 10, 20, 100, 500, or 1000. A higher number of covariates increases accuracy of the results.

The dataset provided in the machine-learning method comprises training samples (i.e., data forming training patterns) each corresponding to a respective (real) patient, and each comprising, for said respective patient, (actual) values of the covariates, as well as time-to-event data, that is, dated information relative to illness states. The number of training samples may be higher than 500, 1000, 5000, 10000, or 20000. A higher number of training samples increases accuracy of the results. The time-to-event data represents an actual chronology of transitions of the patient, for a certain time duration (i.e., follow-up period), from one state of the model to another state of the model, associated with time information (i.e., a date and/or a duration). The time-to-event data thus form ground truth values with which the function is to comply in its output. The dataset may comprise data relative to patients subject to no transition at all, data relative to patients subject to one and only one transition, and data relative to patients subject to several transitions. The time-to-event data may provide the time information in any manner, for example with the actual date of each event, or with a counter (e.g., in days and/or starting from 0).

The input covariates may represent (in the dataset of the machine-learning and/or in the data provided to the use method) patient-wise baseline medical characteristics of the patient. By "baseline", it is meant the beginning of a follow-up period. In other words, the function is trained to be inputted with characteristics of patients acquired (e.g., measured) all with a same date (i.e., at the date of the beginning of the patent follow-up), and to output based thereon a distribution of transition-specific probabilities for a follow-up period thereafter (i.e., starting at that date). In specific, in the dataset, the covariate data may be limited to baseline data, i.e., data dated at the start of the patient-wise chronologies.

The function may be configured to output a distribution of transition-specific probabilities for a fixed follow-up period or time duration. The follow-up period or time duration may be predetermined and defined by the time-to-event data provided in the machine-learnt method. In others words, the follow-up period may be a function of the training time-to-event data. The predetermined follow-up period may for example be any period of time larger than one month, six months, one year, two years, three years, five years, seven years, or ten years. In such a case, the function may be configured to output a distribution of transition-specific probabilities for the fixed follow-up period, or alternatively for a truncated follow-up period shorter than the follow-up period of the dataset.

In examples, substantially all the training samples of the dataset present the same follow-up period (i.e., the period of the time-to-event data is the same for substantially all the patients), and the follow-up period of the trained function may be predetermined to be equal or shorter than said same follow-up period.

Examples of the neural network architecture of the function are now discussed.

The function may comprise a covariate-shared subnetwork and/or a transition-specific subnetwork per transition (e.g., three transition-specific subnetworks in total). In other words, the covariates (e.g., in a unique vector) may be inputted to and processed by one common and single subnetwork of the function. The output of such shared processing may then feed in parallel respective subnetworks for each transition of the multi-state model. This allows high accuracy of the function.

In particular, the covariate-shared subnetwork may comprise (e.g., consist of) a respective fully connected neural network. Additionally or alternatively, at least one (e.g., each) transition-specific subnetwork may comprise (e.g., consist of) a fully connected neural network. A fully connected neural network comprises a series of one or more fully connected layers that connect every neuron in one layer to every neuron in the other layer. Optionally, the covariate-shared fully connected neural network may comprise several fully connected layers. Additionally or alternatively, at least one (e.g., each) transition-specific fully connected neural network may comprise several fully connected layers. Further optionally, the covariate-shared subnetwork may comprise a respective non-linear activation function. Additionally or alternatively, at least one (e.g., each) transition-specific subnetwork may comprise a respective non-linear activation function. The fully connected layers (in particular when multiple) and/or non-linear activation functions allow capturing non-linear dependencies in the covariates, thus achieving high accuracy compared to prior art methods based on a linearity assumption.

The number of fully connected layers and/or the number of neurons of each such fully connected neural network may depend on the number of training samples and/or on the number of covariates. Said number of fully connected layers and/or said number of neurons may be determined by random search, or predetermined. Said number of fully connected layers may for example be equal to 1, to 2, to 3, or higher than 3, and/or said number of neurons may be higher than 10 or 15, and/or below 100, 50 or 30, for example between 15 and 30.

Each transition-specific subnetwork may be followed by a softmax layer or any other probabilistic layer. This allows outputting a value between 0 and 1, interpretable as a probability value or a constant probability density. In specific, when the multi-state model comprises competing transitions, the transition-specific subnetworks of the competing transitions may share a common softmax layer or any other probabilistic layer (e.g., the function may comprise exactly two softmax layers in total). This allows learning the probabilities jointly. Such softmax layers ensure that probability to leave the initial state converges to 1 as time increases (as death is certain, due to any cause), and/or that probability to leave the intermediate state converges to 1 as time increases (as death is certain, again due to any cause, be it the relapse of anything else).

The training may comprise minimizing a loss, for example by a gradient stochastic descent.

The loss function may comprise a likelihood term. The likelihood term may penalize non-fit to the dataset. In other words, the likelihood term rewards, for each training sample, an extent to which the output distribution of transition-specific probabilities makes the ground truth likely. The likelihood term may perform such penalization in any manner, for example by being equal to the negative of a log-likelihood.

In the case of an illness-death model, the log-likelihood may comprise a first sub-term representing a log contribution from the initial state, and a second sub-term representing a log contribution from the intermediate state. The log-likelihood may be equal to the sum of the two terms. The first sub-term may optionally be derived under a time non-homogenous markovian assumption for the transitions from the initial state, and/or the second sub-term may optionally be derived under a time homogenous semi-markovian assumption for the transition(s) from the intermediate state.

The function outputs a distribution of transition-specific probabilities for a set of intervals. In other words, the function outputs a single probability value (i.e., real number between 0 and 1) per each transition of the multi-state model and per each interval of the set of intervals. The set of intervals is a partition of the (e.g., predetermined) follow-up period into time intervals. In other words, the time intervals of the set are successive and non-overlapping, and so as to fully cover the follow-up period.

The number of time intervals and the interval cut-points may be predetermined , for example such that each interval lasts between one week and six months, optionally between two weeks and three months, e.g., about one month. The choice of the number of time intervals may have an impact on the model performance. A regularization term may smoothen (e.g., mitigate) the effect of a non-optimal number of time intervals, which limits risks of overfitting and underfitting. For example, the loss function may further comprise a regularization term that penalizes in weight matrices of the function (e.g., weight matrices of the fully connected layers), first order differences of weights associated with two adjacent time intervals, and/or in bias vectors of the function (e.g., bias vectors of the output layers, such as the softmax layers and/or activation functions), first order differences of biases associated with two adj acent time intervals. In other words, the regularization term penalizes differences between weights respectively biases of the neural network that yield high probabilities of transition in consecutive time intervals.

In the case of a predetermined and fixed follow-up period, the interval cut-points (i.e., boundaries) may be selected so as to be equidistant, or alternatively based on the distribution of transition times.

The loss function may comprise or consist of the sum of the likelihood term (e.g., negative log-likelihood) and the regularization term.

Thus, the methods may extend deep neural networks for an illness-death process. The methods may propose a deep learning architecture which models the probabilities of occurrence of the transitions between the states with no linear assumption. A multi-task neural network may include one subnetwork shared for all the transitions and three transition-specific subnetworks. A new form of the log-likelihood of a piecewise constant illness-death process may be derived. Experiments conducted on a simulated non-linear dataset and on a real dataset of patients with breast cancer are provided later to illustrate the performance of this approach.

The use method is now discussed

The outputted distribution of transition-specific probabilities allows performing any type of medical analysis on the patient with respect to the illness, such as prognosis. Examples of such a type of medical analysis are provided in References 9 and 27. Such type of analysis are known from the field of stochastic processes, and are here applied to healthcare.

The use method may comprise, for one or more states of the model, computing the probability to be in a respective state at a certain time of the follow-up period. Additionally or alternatively, the use method may comprise identifying one or more transition risks based on the distribution of transition-specific probabilities, that is, each a risk that the patient follows a respective transition of the multi-state model (and thus changes states accordingly) during the follow-up period. The use method may in particular comprise identifying relapse risk and/or death risk associate to the patient based on the distribution of transition-specific probabilities for each time interval. The relapse and/or death risk may be any type of data, such as a percentage value. The output of the function is the infinitesimal probabilities of experiencing the respective transitions. The probability of experiencing the transition from the intermediate state to the final state is defined conditionally to the time of entering the intermediate state. The relapse and/or death risk identifying may be automatic, or alternatively manual by the healthcare professional.

Additionally or alternatively, the use method may comprise computing one or more transition-specific cumulative incidence functions (CIF), that is, each for a respective transition of the multi-state model. An example of how to compute such functions is provided later in Section 5.1. The CIF may be relied upon for any type of medical analysis, such as for the above relapse risk and/or death risk identifying.

In examples, the use method may further comprise determining a treatment (patient not yet treated for the illness) or a treatment adaptation (patient already being treated for the illness) based on the outputted distribution of transition-specific probabilities. Additionally or alternatively, the method may determine a follow-up visit (e.g., the method may comprise prescribing a time and/or nature of such a follow-up visit), and/or a surveillance with diagnostic tests (e.g., the method may comprise prescribing a time and/or nature of such diagnostic tests). This determining may be based on any of the above medical analysis. For instance, the risk of cancer relapse may be computed and used to stratify the population in high/intermediate/low risk of relapse. This classification may determine the nature and intensity of peri-surgical or adjuvant therapies. Similarly, in atrial fibrillation, the individual cumulative risk of embolic event may be computed and an anticoagulant therapy may be prescribed above a threshold defined in guidelines.

The use method may comprise displaying at least one (e.g., each) transition-wise CIF, for example in a 2D cross-plot (e.g., with time on the abscissa), and/or for example several (e.g., all) CIFs displayed simultaneously (e.g., on the same screen). Additionally or alternatively, the use method may comprise displaying any graphical representation of the outputted distribution of transition-specific probabilities and/or any graphical representation of the automatically identified (i.e., calculated) relapse and/or death risk.

A specific implementation of the methods is now discussed.

Multi-state models can capture the different patterns of disease evolution. In particular, the illness-death model may be used to follow disease progression from a healthy state to an intermediate state of the disease and to a death-related final state. The specific implementation aims to use those models in order to adapt treatment decisions according to the evolution of the disease. In state-of-the art methods, the risks of transition between the states are modeled via (semi-) Markov processes and transition-specific Cox proportional hazard (P.H.) models. However, the Cox P.H. model makes a linear assumption about the relationship between the covariates and the risk of transiting that showed limitations in many real-world applications. To address this challenge, the specific implementation proposes a neural network architecture (referred to as "example function" in the following) that relaxes the linear Cox P.H. assumption within an illness-death process. The example function employs a multitask architecture and uses a set fully connected subnetworks to capture non-linearity in the data in order to learn the probabilities of transiting between the states. Through simulations, the following sections explore different configurations of the architecture and demonstrate the added value of the model. In comparison to state-of-the-art methods, the example function significantly improves the predictive performance on a simulated dataset, on a real-world dataset in breast cancer.

## 2 | THE ILLNESS-DEATH MODEL

This section introduces the traditional illness-death model, see the work of Andersen et al. in Reference 10 for a complete presentation.

### 2.1 | Notation

Multi-state models are a generalization of survival models when multiple events of interest can occur over time. A multi-state process ($E(t)$, $0 < t \leq +\infty$) is a continuous-time stochastic process that describes the states occupied by a patient over time.

The specific implementation considers an illness-death multi-state process with three states: state 0 is the initial state "Event-free", state 1 is an intermediate state "Relapse", state 2 is an absorbing state "Death". The illness-death process is characterized by three irreversible transitions: from 0 to 1 ($0 \rightarrow 1$), from 0 to 2 ($0 \rightarrow 2$), from 1 to 2 ($1 \rightarrow 2$), where transitions from state 0 are competing, transitions $0 \rightarrow 1$ and $1 \rightarrow 2$ are successive. It assumes that all subjects are in state 0 at time $t = 0$ (i.e., $\mathbb{P}\big(E(0) = 0\big)$).

The evolution of an illness-death process is characterized by three random variables (r.v.) $T_{kl}$, for $(k,) \in \{(0,1), (0,2), (1,2)\}$, associated with each of the three transitions. They represent the transition times from state $k$ to state $l$ ($k \neq l$). Subjects leaving state 0 will enter either state 1 at time $T_{01}$ or state 2 at time $T_{02}$. For subjects entered in state 1 at $T_{01}$, they will enter in state 2 at time $T_{01} + T_{12}$. The process can be summarized by two r.v. $T_0$ and $T_2$. The exit time from state 0

$$T_0 = \inf_{t > 0} \{E(t) \neq 0\} = \min\big(T_{01}, T_{02}\big)$$

together with $D_0 \in \{1, 2\}$ indicates the entered state, and the entry time to state 2

$$T_2 = \inf_{t > 0} \{E(t) = 2\} = T_0 + \mathbb{1}\big\{D_0 = 1\big\} T_{12}$$

characterizes the total survival time.

In clinical settings, the true transition times are partially observed because of right-censoring. Let $C$ be a non-negative censoring r.v. independent of $(T_0, T_2)$ that precludes its observation. Let $\tilde{T}_0 = \min(\tilde{T}_0, C)$ and $\tilde{T}_2 = \min(T_2, C)$ be the observed event times. Together with these times, one may observe the binary labels

$$\delta_{0l} = \mathbb{1}\{D_0 = l,\ T_0 \le C\}\ (l = 1, 2),\ \delta_{12} = \delta_{01}\,\mathbb{1}\{T_2 \le C\}$$

that indicate the status of the transitions, where $\delta_{kl} = 1$ indicates an entry in state $l$ from $k$ and $\delta_{kl} = 0$ indicates a censored transition.

### 2.2 | Model definition

Illness-death processes are traditionally defined through the theory of counting processes (see e.g., Reference 23). In an illness-death model, the observation of process $E$ is equivalent to the observation of the three-variate process $t \mapsto N(t) = (N_{01}(t), N_{02}(t), N_{12}(t))$ where

$$N_{kl}(t) = \mathrm{card}\{0 \le s \le t\ :\ E(s-) = k, E(s) = l\},\ \text{for } (k,l) \in \{(0,1), (0,2), (1,2)\},$$

counts for each pair $(k, l)$ the number of observed transitions from state $k$ to state $l$ in interval $[0, t]$.

The specific implementation defines in addition two predictable processes $Y_0$ and $Y_1$ as

$$Y_k(t) = \mathbb{1}\{E(t-) = k\},\ \text{for } k = 0, 1,\ t > 0,$$

that indicate if the patient is in state $k$ before time $t$.

The three-variate counting process $N$ is conventionally associated with a set of transition intensities, or transition-specific hazard functions,

$$\alpha_{kl}(t) = \lim_{h \to 0} \frac{1}{h} \mathbb{P}\big(E(t+h) = l \mid E(t-) = k\big),\ \text{for } (k,l) \in \{(0,1), (0,2), (1,2)\},$$

that expresses the instantaneous risk to transit from a state $k$ to a state $l$ ($l \ne k$) at time $t + h$, with $h \to 0$ conditionally of being in state $k$ at time $t$-.

Depending on the context, the evolution of the three processes in relation to time can be formulated with different assumptions. In general, the processes associated with each of the three transitions might depend on the time $t$ of arrival in the state (i.e., Markov processes) or on the duration $d$ spent in the current state (i.e., a Semi-Markov processes). The choice of the time scale (see e.g., Reference 3) is an important step in disease modeling because it will induct how the disease will evolve over time. Section 3.1 discusses this choice.

### 2.3 | The log-likelihood

The log-likelihood associated with the observation of a three-dimensional counting process $t \mapsto (t) = (N_{01}(t), N_{02}(t), N_{12}(t))$ on $[0, \tau]$, with $\tau$ the horizon time, is given by the log product of the three independent transition-specific likelihoods:

$$\log \mathscr{L} = \log\big(\mathscr{L}^{0 \to 1} \times \mathscr{L}^{0 \to 2} \times \mathscr{L}^{1 \to 2}\big)$$

with $\mathscr{L}^{0 \to 1}, \mathscr{L}^{0 \to 2}, \mathscr{L}^{1 \to 2}$ the three likelihoods associated with the three transitions. Following the work of Andersen et al. (Reference 10), the log-likelihood corresponding to the observation of a censored trajectory $t \mapsto N(t \wedge C) = N_{01}(t \wedge C), N_{02}(t \wedge C), N_{12}(t \wedge C)$ is given by the equations, for $(k, l) \in (0, 1), (0, 2), (1, 2)$,

$$\log \mathscr{L}^{k \rightarrow l} = \int_0^\tau \log \left( \alpha_{kl}(t) \right) Y_k(t) \mathbb{1}\{C \geq t\} dN_{kl}(t) - \int_0^\tau \alpha_{kl}(t) Y_k(t) \mathbb{1}\{C \geq t\} dt. \quad (1)$$

### 2.4 | The transition-specific Cox P.H. model

The Cox P.H. approach is widely used to evaluate covariates effects on patient's survival. Transition specific Cox P.H. models have been extended to multi-state processes in order to evaluate covariate effects on each transition (see Reference 9). Covariate effects are introduced in the transition intensities of log linear risk function as follows:

$$\alpha_{kl}(t|X) = \alpha_{kl}^0(t) \exp \left( X \beta_{kl} \right)\!\!, \text{ for } (k, l) \in \left\{ (0, 1), (0, 2), (1, 2) \right\},$$

where $\alpha_{kl}^0(t)$ is the baseline transition intensity related to the transition $k \rightarrow l$ (i.e., the underlying hazard when all the covariates are equal to zero) and $\beta_{kl}$ is the vector of regression coefficients to be estimated. To estimate the coefficients $\beta_{kl}$, the log likelihoods in Equation (1) are traditionally used.

### 3 | METHODOLOGY

This section presents the approach to model an illness-death process.

### 3.1 | Functions of interest

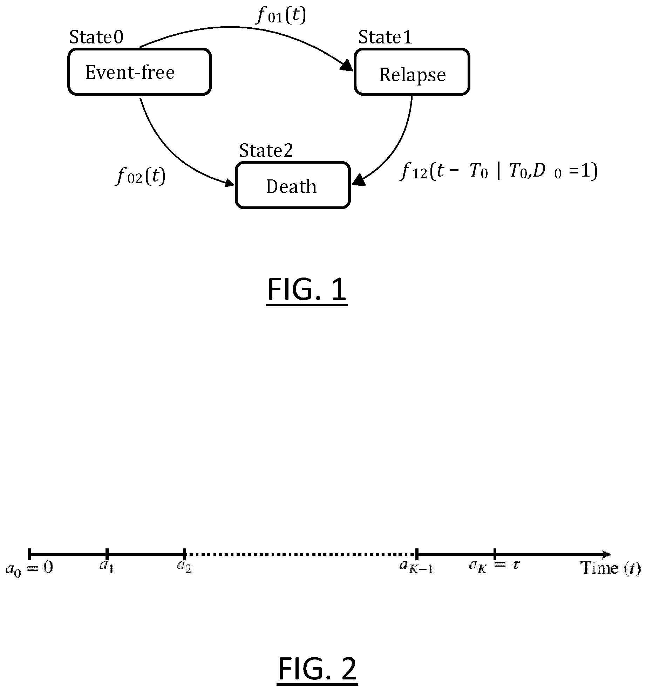

Here, the modeling of the illness-death process may comprise defining the three processes by making specific assumptions on cancer evolution over time (see Reference 24). For the transitions $0 \rightarrow 1$ and $0 \rightarrow 2$, the specific implementation may consider time non-homogeneous markovian processes. For transition $1 \rightarrow 2$, the modeling may comprise performing a time transformation, and considering a time homogeneous semi-markovian process (the probability of transiting from state 1 to state 2 at time $t$ depends only on the duration $t - T_0$ already spent in 1). Wherever convenient, the modeling may comprise using the duration variable $d = t - T_0$ instead of the time variable $t$ for the transition $1 \rightarrow 2$. Under these assumptions, the modeling may aim at modeling the transition probabilities $f_{kl}$ (.) over time. See an illustration of the illness-death process in FIG. 1.

The modeling may comprise defining $f_{01}$ and $f_{02}$ as the infinitesimal probabilities of experiencing respectively transitions $0 \rightarrow 1$ and $0 \rightarrow 2$,

$$f_{0l}(t) = \lim_{h \rightarrow 0} \frac{1}{h} \mathbb{P}\left( E(t+h) = l, E(t-) = 0 \right) = \lim_{h \rightarrow 0} \frac{1}{h} \mathbb{P}\left( t \leq T_0 \leq t+h, D_0 = l \right), \text{ for } l = 1, 2.$$

The modeling may comprise defining $F_{01}$ and $F_{02}$ as their cumulative counterparts such that, for $l = 1, 2$,

$$F_{0l}(t) = \mathbb{P}\left( E(t) \neq l, D_0 = l \right) = \mathbb{P}\left( T_0 \leq t, D_0 = l \right) = \int_0^t f_{0l}(t) dt$$

expresses the probability that a transition $0 \rightarrow l$ occurs on or before time $t$. With these definitions, the modeling may comprise defining

$$f_0(t) = f_{01}(t) + f_{02}(t)$$

as the infinitesimal probability of exiting state 0 at time $t$ and

$$F_0(t) = F_{01}(t) + F_{02}(t)$$

as the probability of having exited state 0 before time $t$.

For the transition $1 \rightarrow 2$, the functions of interest are defined conditionally to $T_0, D_0 = 1$. To simplify the notations, one may drop this conditioning in the definitions. The modeling may comprise defining $f_{12}$ as the infinitesimal probability of experiencing transition $1 \rightarrow 2$ such that for $f_{12}(d) := f_{12} (d \mid T_0, D_0 = 1)$,

$$f_{12}(d) = \lim_{h \to 0} \frac{1}{h} \mathbb{P}\big( E(d+h) = 2, E(d-) = 1 \big) = \lim_{h \to 0} \frac{1}{h} \mathbb{P}\big( d \le T_2 - T_0 \le d+h \big).$$

The modeling may comprise defining $F_{12}$ as its cumulative counterpart such that, for $F_{12}(d) := F_{12} (d \mid T_0, D_0 = 1)$,

$$F_{12}(d) = \mathbb{P}\big( E(d) = 2 \big) = \mathbb{P}\big( T_2 - T_0 \le d \big) = \int_0^d f_{12}(d) dd,$$

expresses the probability that a transition $1 \rightarrow 2$ occurs on or before time $d$ conditionally to $T_0, D_0 = 1$.

$F_{01}$, $F_{02}$ and $F_{12}$ are commonly referred to cumulative incidence functions (CIFs) in the literature (see e.g., Reference 25) and are the main functions of interest to predict. The modeling may comprise formulating the illness-death process through these probabilities. The illness-death processes may also be defined through transition intensities (see e.g., Reference 23) as explained in Section 2. Both formalisms are related.

## 3.2 | A piecewise constant approach

The specific implementation may assume that the transition-specific density probabilities are piecewise constant. This has to be understood as an approximation, but the approximation error can be bounded when the true functions are smooth (see e.g., Reference 26).

First, the modeling may comprise defining $\tau$ as the maximum horizon time window. The modeling may comprise dividing the time axis into $K$ disjoint time intervals: $v_1 = [a_0, a_1), \cdots, v_K = [a_{K-1}, a_K)$, with $a_0 = 0$ and $a_K = \tau$, as illustrated in FIG. 2. For any time $s \in [0, [$, the modeling denotes by $_{(s)}$ the time interval to which s belongs.

Assuming a piecewise constant model allows to consider a non-homogeneous model (see Section 3.1) while retaining the hypothesis of homogeneity within the same time interval. It means that the density probabilities are constant within each interval. Hence, the modeling may comprise expressing $f_{0l}(l = 1, 2)$, $f_{12}$ as step functions such that $f_{0l}(t) = f_{0l}(v_{k(t)})$ and $f_{12}(d) = f_{12}(v_{k(d)})$. Equivalently, since the density probabilities are assumed to be piecewise constant, their corresponding cumulative counterparts $F_{0l}(l = 1, 2)$ and $F_{12}$ are piecewise linear:

$$F_{0l}(t) = \sum_{k=1}^{k(t)-1} f_{0l}(v_k)|v_k| + (t - a_{k(t)-1}) f_{0l}(v_{k(t)}), \qquad (2)$$

$$F_{12}(d) = \sum_{k=1}^{k(d)-1} f_{12}(v_k)|v_k| + (d - a_{k(d)-1}) f_{12}(v_{k(d)}), \qquad (3)$$

where $|v_k|$ is the length of interval $v_k$. In Equations (2) and (3), each duration of exposure during the intervals is taken into account. In comparison, only whether an event occurred or not in a given time interval is taken into account in discrete-time models, disregarding the duration of exposure in the given time interval.

In real clinical data, the r.v. $T_0$, $T_2$ can take values after $\tau$ (a patient can leave state 0 or state 1 after $\tau$). Hence, under the piecewise constant assumption, the following equations are satisfied:

$$\sum_{k=1}^{K} f_0(v_k)|v_k| + 1 - F_0(\tau) = 1,$$

$$\sum_{k=1}^{K} f_{12}(v_k)|v_k| + 1 - F_{12}(\tau) = 1, \qquad (4)$$

where $|v_k|$ is the length of interval $v_k$.

### 3.3 | Data observations

In clinical settings, together with the disease history are observed patient characteristics as $P$-dimensional covariates. Hence, the data consists in the observation of $n$ independent and identically distributed r.v. in $\mathbb{R}^P \times \mathbb{R}_+ \times \{0, 1\} \times \{0, 1\} \times \mathbb{R}_+ \times \{0, 1\}$ such that

$$\mathscr{D}_i = \left\{ X_i, \left( \tilde{T}_0^i, \delta_{01}^i, \delta_{02}^i \right), \left( \tilde{T}_2^i, \delta_{12}^i \right) \right\}_{1 \leq i \leq n},$$

where $X_i = (X_{i1}, \cdots, X_{iP})^T$ is a vector of $P$ covariates observed at baseline. From these observations and for each subject $i$, the specific implementation may aim to estimate the true transition specific density probabilities conditionally to the clinical features $X_i$ in order to predict the individual CIFs defined in Equations (2) and (3). To take into account the influence of covariates on the functions of interest, the specific implementation may denote the density probabilities as $f_0(.|X_i)$ ($l = 1, 2$), $f_{12}(.|X_i)$ and their cumulative counterpart as $F_0(.|X_i)$ ($l = 1, 2$), $F_{12}(.|X_i)$.

### 3.4 | Rewriting of the log-likelihood

Under the assumption of a piecewise constant model, the modeling may comprise rewriting the log-likelihood of a piecewise constant illness-death process. The conventional illness-death log-likelihood defined in Equation (1) can be rewritten in terms of the density probability functions introduced in the Section 3.1.

The modeling may comprise defining the log-likelihood $\ell = \log \mathscr{L}$ by dividing the contributions in two distinct parts:

$$\ell = \frac{1}{n} \sum_{i=1}^{n} \left[ \ell_0^i + \ell_1^i \right]. \qquad (5)$$

where $\ell_0^i$ is the log contribution of patient $i$ from state 0. From state 0, patient $i$ with an event a $\tilde{T}_0^i$ can contribute in three ways. Patient $i$ (i) can experience a transition $0 \rightarrow 1$; (ii) can experience a transition $0 \rightarrow 2$; (iii) can be censored at $\tilde{T}_0^i$. Thus $\ell_0^i$ is given by

$$\ell_0^i = \sum_{l=1,2} \left\{ \delta_{0l}^i \log \left( f_{0l} \left( v_{k(\tilde{T}_0^i)} \,|\, X_i \right) \right) \right\} + \left( 1 - (\delta_{01}^i + \delta_{02}^i) \right) \log \left( 1 - F_0(\tilde{T}_0^i \,|\, X_i) \right).$$

On the other hand, $\ell_1^i$ is the log contribution of patient $i$ from the time he has entered state 1 (only for $i$ such that $\delta_{01}^i = 1$).

Following the previous reasoning, patient $i$ can contribute in two ways at time $\tilde{T}_2^i - \tilde{T}_0^i$. Patient $i$ (i) can experience a transition $1 \rightarrow 2$; (ii) can be censored. Thus $\ell_1^i$ is given by

$$\ell_1^i = \delta_{01}^i \delta_{12}^i \log \left( f_{12} \left( v_{k(\tilde{T}_2^i - \tilde{T}_0^i)} \,|\, X_i \right) \right) + \delta_{01}^i (1 - \delta_{12}^i) \log \left( 1 - F_{12}(\tilde{T}_2^i - \tilde{T}_0^i \,|\, X_i) \right).$$

The log-likelihood in Equation (5) has been derived under a time non-homogeneous markovian assumption for transition $0 \rightarrow 1$ and $0 \rightarrow 2$, and a time homogeneous semi-markovian assumption for transition $1 \rightarrow 2$. Depending on the application, time assumptions can be reformulated, and this would change the log likelihood above.

### 4 | DESCRIPTION OF THE EXAMPLE FUNCTION

This section describes how the specific implementation may use a new deep learning approach to parametrize the step probability functions $f_{01}, f_{02}, f_{12}$ over the interval $[0, \tau]$. The specific implementation may define a deep learning architecture that is parameterized to model the relationship between covariates and transition probabilities. Unlike classical methods (such as in References 9 and 27), the example function is divided into transition-specific tasks and uses non-linear activation functions to capture nonlinearity between covariates and transition probabilities. The specific implementation proposes a loss function that encompasses the negative log-likelihood of Equation (5) and that is tuned to smooth the effect of a non-optimal value of $K$ (i.e., the number of time intervals), in order to minimize the risk of over-fitting. In addition, the specific implementation may propose two methods to select the interval cut-points.

### 4.1 | Network architecture

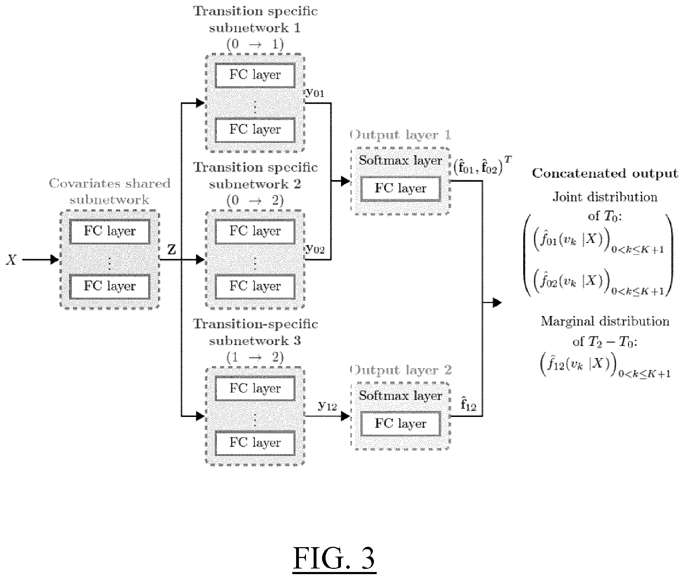

FIG. 3 illustrates an example of architecture FIG. of the example function. The architecture of the example function comprises three task-specific subnetworks that are related to the three transitions of an illness-death process. Multi-task learning is performed with hard parameter sharing (see Reference 28) in order to extract common and specific patterns from the patient's characteristics (i.e., the baseline covariates). It is composed of a first subnetwork shared between the covariates (coordinates of matrix $X$) three transitions and of three transition-specific subnetworks. Two different softmax output layers are used to transform the transition-specific subnetworks outputs into time-dependent probabilities. One softmax layer is related to the exit from state 0 (i.e., the transitions $0 \to 1$ and $0 \to 2$), the other to the exit from state 1 (i.e., the transition $1 \to 2$). The first subnetwork and the transition-specific subnetworks are fully connected (FC), and each fully connected subnetwork may comprise several FC layers as shown on the figure.

### Input layer

The input layer may be composed of the matrix $X$ of $P$ baseline covariates for the $n$ individuals (offline), or a vector $X$ of $P$ baseline covariates for one individual (online).

### Covariates shared subnetwork

The shared subnetwork may take as input the *input layer X* and contain $L$ fully connected hidden layers with $l$ units. Its output may be a vector $\mathbf{z} = g^{\text{input}}(X)$ in $\mathbb{R}^l$ that captures shared patterns between the three transitions ($g^{\text{input}}$ is a non-linear activation function).

### Transition-specific subnetworks

Each transition-specific subnetwork may take as input the output of the shared network $\mathbf{z}$ and contains $L^{kl}$ fully connected hidden layers with $l^{kl}$ units. Its output may be a vector $\mathbf{y}_{kl} = g^{kl}(\mathbf{z})$, that is a transition-specific transformation of the shared features ($g^{kl}$ is a non-linear activation function). Given the unbalanced number of observations for the three transitions that may exist in real data, the range of model complexity may be different for each of the three transitions. If the architecture is too complex for a transition, the model might be poorly generalist on new data (over-fitting). If the architecture is not complex enough for a transition, the model might not capture all the information in the data (under-fitting). To find the best configuration that will result in the best model performance for each of the three transitions, the specific implementation may set the structure of each subnetwork independently. Hence, a transition with a high number of observations (often the transition $0 \to 1$) may support more hidden layers than a transition with less observations (often the transition $0 \to 2$).

### Probabilistic output layers

The output of the network may be composed of two probabilistic layers that map the transition-specific outcomes $\mathbf{y}_{kl}$ into time-dependent probabilities.

Under the constraint of Equation (4), the example function may consider a supplementary interval $v_{K+1} = [\tau, +\infty)$. Consequently, the example function may fulfill the constraint by defining $1 - F_0(\tau) = f_0(v_{K+1})$, $1 - F_{12}(\tau) = f_{12}(v_{K+1})$. Hence, each output of the network may be a fully connected layer which (i) uses a linear activation function $g^{\text{linear}}$ to transform $(\mathbf{y}_{01}, \mathbf{y}_{02})^T$ (resp. $\mathbf{y}_{12}$) into a vector $\phi_0 = (\phi_{01}, \phi_{02})^T \in \mathbb{R}^{2(K+1)}$ (resp. a vector $\phi_{12} \in \mathbb{R}^{K+1}$), and then (ii) uses a weighted softmax activation function $\sigma_0$ (resp. $\sigma_2$) to transform $\phi_0$ (resp. $\phi_{12}$) into probabilities and provide an estimation of $f_{01}$, $f_{02}$ (resp. $f_{12}$) in each time interval. Thus, the output layers may be characterized by the vectors

$$\hat{\mathbf{f}}_0 = \left(\hat{\mathbf{f}}_{01}, \hat{\mathbf{f}}_{02}\right)^T = \sigma_0\left(g^{\text{linear}}\left((\mathbf{y}_{01}, \mathbf{y}_{02})^T\right)\right) = \sigma_0\left((\phi_{01}, \phi_{02})^T\right),$$

$$\hat{\mathbf{f}}_{12} = \sigma_2\left(g^{\text{linear}}\left(\mathbf{y}_{12}\right)\right) = \sigma_2\left(\phi_{12}\right),$$

where

$$\hat{\mathbf{f}}_{kl} = \left( \hat{f}_{kl}(v_k \mid X) \right)_{0 < k \le K+1},$$

and $\sigma_0$, $\sigma_2$ are two softmax functions weighted by the length of the time intervals such that

$$\hat{f}_{0l}(v_k \mid X) = \frac{\exp\left[\phi_{0l}^k(X)\right]}{\sum_{j=1}^{K+1} \left( \exp\left[\phi_{01}^j(X)\right] + \exp\left[\phi_{02}^j(X)\right] \right) \left| v_j \right|}, \text{ for } l = 1, 2,$$

$$\hat{f}_{12}(v_k \mid X) = \frac{\exp\left[\phi_{12}^k(X)\right]}{\sum_{j=1}^{K+1} \exp\left[\phi_{12}^j(X)\right] \left| v_j \right|}.$$

### 4.2 | Loss function and mitigation of the number of time intervals effect via regularization

To learn the example function parameters, the training may comprise minimizing a total loss function,

$$\ell_{total} = -\ell^{K+1} + P_\lambda, \qquad (6)$$

that sums the negative log-likelihood and a regularization term.

The first term $-\ell^{K+1}$ is a revising of the negative log-likelihood $-\ell$ defined in Equation (5) under the constraint of Equation (4) (i.e., considering a supplementary interval $K + 1$).

The second term $P_\lambda$ is a regularization term related to $\ell^{K+1}$ allowing to smooth the effect of a non-optimal number of time intervals (i.e., a non optimal value for $K$). The choice of $K$ may have an impact on the performance: the number of nodes grows with $K$, which might cause over-fitting (for a large value of $K$) or under-fitting. One option may be to choose a large $K$ while preventing over-fitting by using an L1 regularization over weights in the output layer. Another option may be to fix a small value for $K$ in order to reduce the size of the output layers as much as possible. As yet another option, an automatic selection of $K$ can be fixed by applying a temporal smoothing technique. The training may comprise applying a temporal smoothness constraint by penalizing, in the weight matrices (resp. the bias vectors) of the output layers, the first order differences of the weights (resp. the bias) associated with two adjacent time intervals. Let us consider $W = (W^1, W^{12})$, $B = (B^1, B^{12})$ the weight and bias parameters associated with the two output layers, with $W^1 = (W^{01}, W^{02}) \in \mathbb{R}^{(l^{01}+l^{02}) \times 2(K+1)}$, $W^{12} \in \mathbb{R}^{l^{12} \times (K+1)}$ and $B^1 = (B^{01}, B^{02})^T \in \mathbb{R}^{2(K+1)}$, $B^{12} \in \mathbb{R}^{K+1}$. For $k = 1 \dots, K$, the training may comprise computing

$$\Delta_{w_{j,k}^{kl}} = w_{j,k+1}^{kl} - w_{j,k}^{kl}, \quad \Delta_{b_k^{kl}} = b_{k+1}^{kl} - b_k^{kl},$$

the weight and bias differences associated with the transition $k \to l$, neuron $j$ and adjacent time intervals $v_k$, $v_{k+1}$. Then the penalty term of the loss function in Equation (6) has the form

$$P_\lambda(B, W) = \sum_{kl} \left( \lambda_w^{kl} \sum_{j=1}^{l^{kl}} \sum_{k=1}^{K} \left| \Delta_{w_{j,k}^{kl}} \right| + \lambda_b^{kl} \sum_{j=1}^{l^{kl}} \sum_{k=1}^{K} \left| \Delta_{b_{j,k}^{kl}} \right| \right),$$

where $\lambda_w^{kl}$ and $\lambda_b^{kl}$ are transition-specific positive constants determining the amount of smoothing to be applied for each transition. For $\lambda_w^{kl} \to +\infty$ (respectively $\lambda_b^{kl} \to +\infty$), all differences will be set to zero resulting in constant weights (respectively constant bias). This regularization term allows to minimize the risk of over-fitting, for the three transitions independently, for larger values of $K$.

### 4.3 | Selection of the interval cut-points $a_k$'s

Under the piecewise constant assumption (see Section 3.2), the definition of the density probabilities may be such that time is on the form $0 = a_0 < a_1 < \cdots < a_K = \tau$. Hence, the specific implementation may comprise performing a division of the time scale and the selection of the interval cut-points $a_k$'s ($k = 1, \cdots, K$). On the one hand, the specific implementation may comprise selecting sufficiently wide interval cut-points to retain enough information in each interval (i.e., keeping a sufficient number of observed transitions within each interval). On the other hand, the specific implementation may comprise selecting sufficiently narrow cut-points to ensure that significant temporal changes in the density probabilities can be identified.

To select the cut-points, one option is to chose $K$ equidistant cut-points in $[0, \tau]$ (i.e., uniform intervals each of length $K/\tau$). An alternative option is to select the cut-points based on the distribution of the transition times. This approach yields different cut-points $a_k^1$, $a_k^2$ for the two output layers of the example function. In that case, for the first output layer related to the transitions $0 \to l$ ($l = 1, 2$), the specific implementation may comprise selecting the cut-points by estimating the $K$ quantiles $q_k^1$ $(k = 1, \cdots, K)$ of the marginal distribution of the sojourn duration in state 0 (i.e., $1 - F_0(.)$). In the same way, for the second output layer related to the transition $1 \to 2$, the specific implementation may comprise selecting the cut-points based on the $K$ quantiles $q_k^2$ $(k = 1, \cdots, K)$ of the distribution of the sojourn duration in state 1 among patients at risk (i.e., $1 - F_{12}(.)$). The quantiles $q_k^1, q_k^2$ (for $k = 1, \cdots, K$) verify:

$$1 = 1 - F_0(0) = q_0^1 > q_1^1 > \cdots > q_K^1 = 1 - F_0(\tau),$$

$$1 = 1 - F_{12}(0) = q_0^2 > q_1^2 > \cdots > q_K^2 = 1 - F_{12}(\tau),$$

and can be found by estimating the duration distributions $F_0$ and $F_{12}$ via the non-parametric Aalen-Johansen estimator (Reference 32). This approach ensures that each time interval has the same decrease in the duration distribution estimates, such that

$$q_{k+1}^1 - q_k^1 = \left(1 - F_0(\tau)\right)/K,$$

$$q_{k+1}^2 - q_k^2 = \left(1 - F_{12}(\tau)\right)/K.$$

Finally, by denoting $\hat{F}_0^{AJ}$, $\hat{F}_{12}^{AJ}$ the Aalen-Johansen estimators of $F_0, F_{12}$, the interval cut-points may be found by solving

$$1 - \hat{F}_0^{\mathrm{AJ}}(a_k^1) = q_k^1,$$

$$1 - \hat{F}_{12}^{\mathrm{AJ}}(a_k^2) = q_k^2.$$

A similar approach may be used in the case of a unique event.

### 5 | PREDICTION TASK AND BENCHMARK

### 5.1 | Prediction of the individual CIFs

This subsection defines the predictions of interest according to the time scales defined below. From the output of the network (i.e., the step functions $\hat{f}_{0l}(.|X)$ ($l = 1, 2$), $f_{12}$ $(.| X)$), one may derive the estimation of the CIFs.

For a new patient $j$ with the baseline covariates $X_j$, one may note the estimated CIFs, derived from Equations (2) and (3), as $\hat{F}_{0l}(.| X_j)$ ($l = 1, 2$), $\hat{F}_{12}$ $(.| X_j)$, such that:

$$\hat{F}_{0l}\left(t|X_j\right) = \sum_{k=1}^{k(t)-1} \hat{f}_{0l}\left(v_k|X_j\right)|v_k| + (t - a_{k(t)-1})\hat{f}_{0l}\left(v_{k(t)}|X_j\right) \text{ for } l = 1, 2,$$

$$\hat{F}_{12}\left(d|X_j\right) = \sum_{k=1}^{k(d)-1} \hat{f}_{12}\left(v_k|X_j\right)|v_k| + (d - a_{k(d)-1})\hat{f}_{12}\left(v_{k(d)}|X_j\right).$$

The estimated CIFs are used to assess the predictive performance of the example function.

It is to be noted that the use method of the specific implementation may in examples comprise computing and/or displaying such CIFs.

### 5.2 | Predictive evaluation criteria

In event history analysis, commonly used performance measures are the time-dependent AUC (for discrimination, see Reference 33) and the time dependent Brier score (BS, see Reference 34) (for calibration). On the basis of the transition-specific time properties defined in Section 3.1, one may adapt the definitions of the time-dependent AUC and the time-dependent Brier score.

To evaluate predictive performances related to transitions $0 \to 1$ and $0 \to 2$, all the patients are considered. For the transition $1 \to 2$, predictions of interest are formulated conditionally to be in state 1 at time $T_0$ and from the duration $t$ - $T_0$. Hence only the patients at risk of experiencing the transition are considered (i.e., only the patients who have already experienced a transition $0 \to 1$).

For two patients $i$ and $j$, the transition-specific time-dependent AUC measures the probability that a patient $i$ who experienced the transition $kl$ before time $t$ has greater probability of occurrence of the transition than a patient $j$ who has survived to the transition. For, on the one hand the transitions $0 \to 1$, $0 \to 2$, and on the other hand the transition $1 \to 2$, one may define

$$\mathrm{AUC}^{0l}(t) = \mathbb{P}\left( F_{0l}\left(t|X_i\right) > F_{0l}\left(t|X_j\right) \mid T_0^i \le t, \ T_0^j > t, \ D_0^i = l\right),$$

for $l$ = 1, 2,

$$\mathrm{AUC}^{12}(d) = \mathbb{P}\left( F_{12}\left(d|X_i\right) > F_{12}\left(d|X_j\right) \mid T_2^i - T_0^i \le d, \ T_2^j - T_0^j > d, D_0^i = 1, \ D_0^j = 1\right).$$

Commonly, the AUC is defined as the integration of the ROC curve opposing specificity (Sp) and sensitivity (Se).

The transition-specific time-dependent Brier score measures the difference between the predicted probability of occurrence of the transition at time t and the status of the transition, such that:

$$\mathrm{BS}^{0l}(t) = \frac{1}{n}\sum_{i=1}^{n}\left[\mathbb{1}\left\{T_0^i > t\right\} - F_{0l}\left(t|X_i\right)\right]^2 \text{ for } l = 1, 2,$$

$$\mathrm{BS}^{12}(d) = \frac{1}{n_{12}}\sum_{i: D_0^i=1}\left[\mathbb{1}\left\{T_2^i - T_0^i > d\right\} - F_{12}\left(d|X_i\right)\right]^2,$$

where $n_{12}$ is the number of patients at risk for transition $1 \to 2$.

Several estimators have been developed taking into account the loss of information due to censoring by using the inverse probability of censoring weight (IPCW) methods. To evaluate predictive performances related to transitions $0 \to 1$ and $0 \to 2$, one may use classical IPCW estimators already developed in the literature (see e.g., References 33 or 34). For the transition $1 \to 2$, one may rewrite the classical estimators under the semi-markovian assumption considering only the patients at risk for experiencing the transition.

The time-dependent AUC and the time-dependent BS can be extended to the interval $]0, \tau]$ by computing respectively the integrated AUC (iAUC) and the integrated Brier score (iBS) as follows:

$$\mathrm{iAUC}^{kl} = \frac{1}{\tau} \int_0^\tau \mathrm{AUC}^{kl}(t)dt, \ \mathrm{iBS}^{kl} = \frac{1}{\tau} \int_0^\tau \mathrm{BS}^{kl}(t)dt.$$

### 5.3 | Benchmark and validation

Predictive performances of the example function in predicting the CIFs are compared in terms of discrimination (with the iAUC) and calibration (with the iBS) with two state-of-the-art statistical methods: the multi-state Cox P.H model (msCox), that is defined in Section 2.4, from the R library mstate (Reference 9) and a spline-based version of the Cox multi-state model (msSplineCox) from the R library flexsurv (Reference 35). The example function is also compared with a simplified linear version (Linear example function). The simplified linear version has no covariate-shared subnetwork, and no transition-specific subnetworks.

Two sets of experiments were performed on (1) a simulated dataset and on (2) one real clinical dataset. For the two sets of experiments, one may score predictive performances of the methods through internal validation (see Reference 36) by randomly splitting $M = 50$ times each dataset $\mathscr{D}$ into a training/testing dataset $\mathscr{D}_m^{\mathrm{train}}$ (68% for training / 12% for early stopping) and a validation dataset $\mathscr{D}_m^{\mathrm{val}}$ (20%) (for m=1,...,M). For each split of the dataset, one may perform $R = 20$ times a random hyper-parameters search (see Reference 37) and one may choose the set of hyper-parameters associated with the best iAUC (averaged across the three transitions) on the held-out validation sets.

The example function may be implemented in Python within a Tensorflow environment. The example function may use at least one of following deep learning techniques: $L_2$ regularized layers to avoid over-fitting, $L_1$ regularized output layers, Xavier Gaussian initialization schemes, Adam optimizer, mini-batch learning, learning rate weight decay and early stopping. The example function may be optimized in two steps including the hyper-parameters optimization and the parameters optimization. Hyper-parameters of the example function may include at least one of number of nodes and hidden layers in each subnetwork, regularization penalty parameters, output-specific penalization parameters and activation functions for each subnetwork. Hyper-parameters may be tuned using Random Search. For each hyper-parameter, a discrete space of search may be fixed by manual search. For each model, the sets of search are adjusted according to the data set in input.

The median ($\pm$ standard deviation (sd)) iAUC (higher the better) and iBS (lower the better) is compared on the validation sets. Performances of the example function over the three other methods is statistically compared using a bilateral Wilcoxon (Reference 38) signed rank test. In the results, · indicates a p-value less than 0.1, † less than 0.05, ‡ less than 0.01, * less than 0.001.

## 6 | EXPERIMENTS ON SIMULATED DATA SETS

Through simulations, first illustrated are the effects of the example function parameterization on the predictive performance and secondly is compared the example function performance with other methods cited above.

### 6.1 | Data simulation

For the first set of experiments, the simulations may comprise generating continuous-time illness-death data. The horizon-time window is fixed at $\tau = 100$ and generate three datasets $\mathscr{D}_{\mathrm{non-lin.}}^{5000}$, $\mathscr{D}_{\mathrm{non-lin.}}^{20000}$, $\mathscr{D}_{\mathrm{non-lin.}}^{60000}$ of size n = 5000, 20000, 60000, respectively with training sets of size $n_{\mathrm{tr}} = 3400, 13600, 40800$ (i.e., 80% of the datasets) and validation sets $n_{\mathrm{val}}$ of 20%. For each observation $i$ ($1 \leq i \leq n$) the simulations may comprise generating four 2-dimensional baseline variables, each drawn from a multivariate Gaussian distribution with mean 0 and a matrix of variance covariance $\Sigma_p$:

$$X_i = \left( X_i^{(1)}, X_i^{(2)}, X_i^{(3)}, X_i^{(4)} \right)^T \ \mathrm{with} \ X_i^{(p)} \in \mathbb{R}^2 \sim \mathscr{N}\left(0, \Sigma_p\right), \ 1 \leq p \leq 4,$$

where the entries of the matrix $\Sigma_p^{1/2}$ are simulated from i.i.d. uniform variables on [0, 1].

The simulations aim to generate the processes $T_0$ (together with $D_0$) and $T_2$, such that the illness-death times $T_{kl}$, for $(k, l) \in \{(01), (02), (12)\}$, are simulated through Cox transition-specific hazard functions:

$$T_{kl}^i \sim \alpha_{kl}(t|X_i) = \alpha_{kl}^0(t) \exp\left(g_{kl}\left(X_i, \beta_{kl}\right)\right)$$

where $g_{kl}(.)$ is a transition-specific risk function, $\beta_{kl} = (\beta_{kl}^{(1)}, \beta_{kl}^{(2)}, \beta_{kl}^{(3)}, \beta_{kl}^{(4)})^T$ with $\beta_{kl}^{(p)} \in \mathbb{R}^2$ for $1 \leq p \leq 4$ are fixed effect coefficients, and $\alpha_{kl}^0(.)$ is the baseline hazard function. The three baseline hazard functions are generated as follows: $\alpha_{kl}^0(.) \sim$ Weibull (scale = 0.01, shape = 1.2).

The transition-specific risk functions are set to be non-linear using quadratic functions, as

$$g_{01}\left(X_i, \beta_{01}\right) = \left(X_i^{(1)}\beta_{01}^{(1)} + X_i^{(2)}\beta_{01}^{(2)}\right)^2,$$

$$g_{02}\left(X_i, \beta_{02}\right) = \left(X_i^{(2)}\beta_{02}^{(2)} + X_i^{(3)}\beta_{02}^{(3)}\right)^2, \qquad (7)$$

$$g_{12}\left(X_i, \beta_{12}\right) = \left(X_i^{(3)}\beta_{12}^{(3)} + X_i^{(4)}\beta_{12}^{(4)}\right)^2.$$

Arbitrary values are fixed for the fixed effects coefficients. Hence, in this simulation scheme, the Cox's linear assumption does not hold anymore.

From the simulated $T_{kl}$, the simulation of the processes $T_0$ and $T_2$ has to respect constraints of the model (i.e. $T_{01}$ and $T_{02}$ are competing, $T_{01}$ and $T_{12}$ are recurrent) in order to generate an identifiable Cox model . $r = 30\%$ is fixed such that 30% of patients from state 0 are censored, and 30% of patients at risk for transition $1 \rightarrow 2$ are censored from state 1, see Table 1. Table 1 shows the descriptive statistics on the number of (No.) observations in the simulated datasets.

**TABLE 1:**

| Data set | No. observations (%) | | | | | Total |
|---|---|---|---|---|---|---|
| | $0 \rightarrow 1$ | $0 \rightarrow 2$ | $0 \rightarrow$ cens. | $1 \rightarrow 2$ | $1 \rightarrow$ cens. | |
| $\mathscr{D}_{\text{non-lin.}}^{5000}$ | 1817 (36%) | 1683 (34%) | 1500 (30%) | 1272 (70%*) | 545 (30%*) | 5000 |
| $\mathscr{D}_{\text{non-lin.}}^{20000}$ | 7234 (36%) | 6766 (34%) | 6000 (30%) | 5064 (70%*) | 2170 (30%*) | 20000 |
| $\mathscr{D}_{\text{non-lin.}}^{60000}$ | 21729 (36%) | 20271 (34%) | 18000 (30%) | 15211 (70%*) | 6518 (30%*) | 60000 |
| * among patients at risk | | | | | | |

## 6.2 | Simulation study

### 6.2.1 | Understanding the effect of *K* and n

To get an improved understanding of the methodologies discussed in Sections 4.2 and 4.3, a simulation study is carried out, in which the size n of the datasets is varied, the number *K* of time intervals used for the piecewise approximations and the selection methods of the interval cut-points.

For evaluation, the internal validation procedure described in Section 5.3 is used. The evaluation uses the iAUC and iBS measures, in addition to the transition-specific integrated Mean Absolute Error (iMAE$_{kl}$) between the estimated CIFs $\hat{F}_{kl}(.| X_i)$ and the true CIFs $F_{kl}(.| X_i)$:

$$\text{iMAE}_{kl} = \frac{1}{\tau}\int_0^\tau \frac{1}{n_{\text{val}}}\sum_{i=1}^{n_{\text{val}}} \left|\hat{F}_{kl}(t|X_i) - F_{kl}(t|X_i)\right|, \text{ for } (k,l) \in \left\{(0,1),(0,2),(1,2)\right\},$$

where $n_{\text{val}}$ is the number of subjects in the validation sets.

For the discretization of the time scale in the three datasets, the number *K* of time intervals is varied in *K* = 25, 50, 100, 200. The simulation study comprises the application of two methods to select the interval cut-points with either equidistant cut-points (uniform) or cut-points obtained with the Aalen-Johansen quantiles (quantilesAJ).

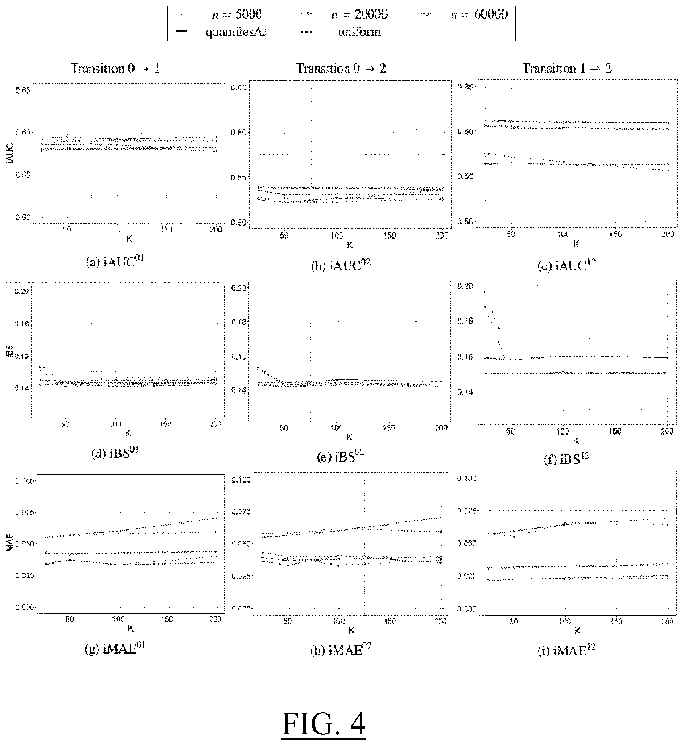

FIG. 4 illustrates an example of a plot of the transition-specific validated criteria (iAUCs, iBSs, iMAEs) of the cut-points selection methods versus the values of $K$ and $n$. In FIG. 4, the full lines represent the method quantilesAJ, while the dotted lines represent the method uniform. The AUC and BS measures are integrated at all the 4 equidistant time points in [0,] (i.e., at times $t$ = 4, 8, 12, ..., 96, for computational cost reasons). The MAE measure are integrated at all the 100 discrete time points in [0,] (i.e., at times $t$ = 1, 2, 3 ..., 100). FIG. 4 shows that the selection methods of the interval cut-points give similar performances in terms of iAUC and iMAE. However, in term of iBS, the uniform selection method give slightly poorer performance for a smaller value of $K$ (for $K$ = 25) than the quantilesAJ selection method. In terms of iBS and iMAE for the three transitions, one may see that a larger value of $n$ increases the performances for all the values of $K$. In addition, for the transition $1 \rightarrow 2$, a larger value of $n$ increases the performance in terms of iAUC as well. However, for the transitions $0 \rightarrow 1$ and $0 \rightarrow 2$, the differences in performances between the values of n are very small.

### 6.2.2 | Understanding the role of $P_\lambda$

To get an improved understanding of the role of the regularization term $P_\lambda$ in the loss function in Equation (6), a second study on the simulated dataset $\mathscr{D}^{5000}_{\text{non-lin.}}$ is carried out, in which the effect of $P_\lambda$, the number $K$ of time intervals ($K$ = 25, 50, 100, 200) and the selection methods of the interval cut-points (uniform, quantilesAJ) is varied.

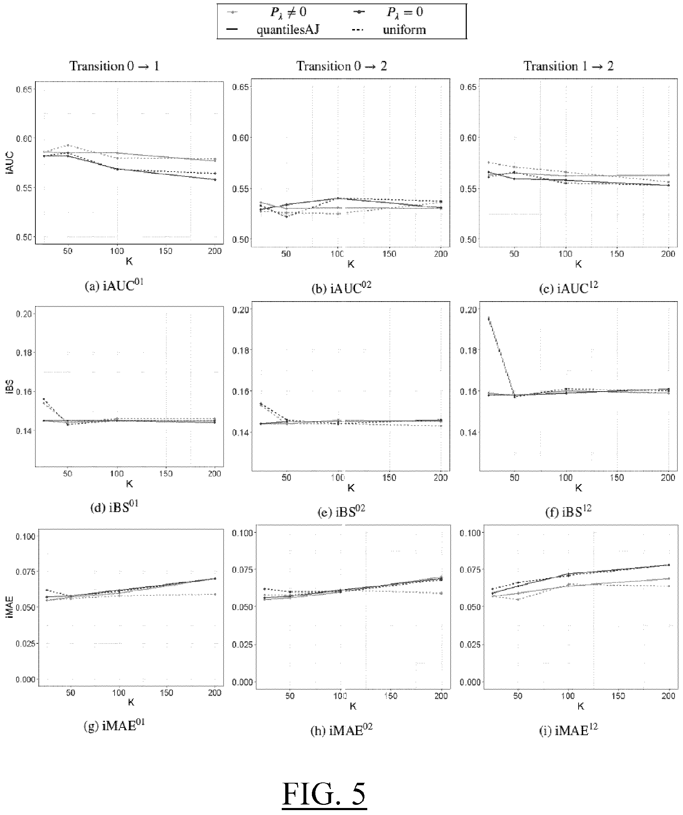

FIG. 5 illustrates an example of a plot of the transition-specific validated criteria (iAUCs, iBSs, iMAEs) versus the values of $K$. FIG. 5 illustrates the effect of the regularization term $P_\lambda$ in the loss function in Equation (6) on the simulated dataset $\mathscr{D}^{5000}_{\text{non-lin.}}$: median iAUCs, iBSs and iMAEs per transition, for each specified value of $K$. The full lines represent the method quantilesAJ, while the dotted lines represent the method uniform. The red color represents the performance of the example function with $P_\lambda \neq 0$, while the brown color represents the performance of the example function with $P_\lambda = 0$. In terms of iAUCs, the regularized model (i.e., with $P_\lambda \neq 0$) outperforms the unregularized model (i.e., with $P_\lambda = 0$) for all the values of $K$ for the transitions $0 \rightarrow 1$ and $1 \rightarrow 2$. In terms of iBSs, the regularized and the unregularized models display similar performances for all the values of $K$ for the three transitions. In terms of iMAEs, the regularized model outperforms the unregularized model for all the values of $K$ for the three transitions. Subsequently, the plot shows an interaction between the value of $K$ and the effect of the regularization regarding the iAUCs and the iMAEs. For the smaller value of $K$ (i.e., $K$ = 25), the regularized and the unregularized models are equivalent.

For the larger value of $K$ (i.e., $K$ = 200), the regularized model outperforms the unregularized model. Thus, it is evident that when the value of $K$ increases, the performance of the unregularized model decreases while the performance of the regularized model is stable. Hence, the regularization term $P_\lambda$ allows to mitigate the effect of a too large value of $K$ on the example function performance.

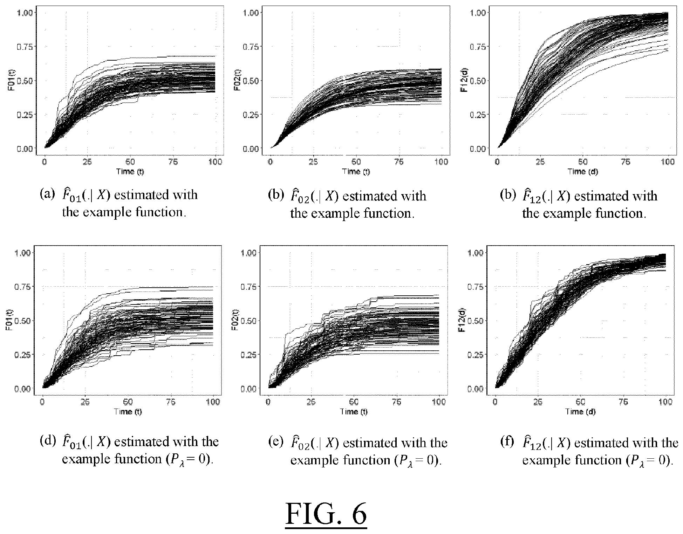

In addition, the role of the regularization term $P_\lambda$ is to provide smooth estimates of the density probability functions. Indeed, it may be reasonable to assume that large temporal variations in the density functions over successive time intervals should be smoothed. For a too large value of $K$, it may result in the emergence of high jumps when computing the CIFs. This problem can be fixed by applying a temporal smoothing method as done by $P_\lambda$. To illustrate the point, the simulated dataset $\mathscr{D}^{5000}_{\text{non-lin.}}$ is used to generate 100 additional observations. The CIFs of the additional observations are estimated with the example function in the case of $P_\lambda = 0$ (unregularized model) and in the case of $P_\lambda \neq 0$ (regularized model). FIG. 6 illustrates the results per transition and shows estimated $\hat{F}_{kl}(.|X)$ in $[0, \tau]$ on the dataset $\mathscr{D}^{5000}_{\text{non-lin.}}$ for 100 additional simulated patients $((k, l) \in \{(01), (02), (12)\})$. For the transitions $0 \rightarrow 1$ and $0 \rightarrow 2$, the regularization term smoothens the estimates when high jumps are estimated between adjacent time intervals. For the transition $1 \rightarrow 2$, estimates of the example function with $P_\lambda = 0$ result in a loss of the variability that is corrected by setting $P_\lambda \neq 0$.

### 6.3 | Benchmark

The non-linear simulated dataset $\mathscr{D}^{5000}_{\text{non-lin.}}$ is used to compare the predictive performance of the example function with the state-of-the-art methods (see Section 5.3). To benchmark the example function, $K$ = 100 is set to divide the time scale into intervals of uniform length.

Table 2 shows the integrated predictive performances. In particular, table 2 shows the predictive performance (median $\pm$ sd) on the validation sets (internal validation) for the non-linear simulated dataset $\mathscr{D}^{5000}_{\text{non-lin.}}$, with $K$ = 100 and a uniform subdivision of the time scale. "Linear ex. func." refers to the Linear example function and "Example function" refers to the example function. In this simulated dataset, the Cox's linear assumption does not hold anymore. Consequently, as expected, the example function significantly outperforms msCox and msSplineCox with a p-value less than 0.001 for the three transitions in terms of AUCs and Brier scores (except for the transition $1 \rightarrow 2$ where msCox outperforms the example function but with no statistical difference). The example function significantly outperforms the linear version function as well. Moreover, the Linear example function outperforms msCox and msSplineCox. This illustrates the effect of a deep learning approach as compared with a statistical approach to model an illness-death process. The predictive performances of the example function may also be evaluated on a linear simulated dataset. With the linear simulated dataset, the example function displays significant better iBSs (with a p-values less than 0:001) for transitions $0 \rightarrow 1$ and 0.2, and equivalent iBS for the transition $1 \rightarrow 2$.

**TABLE 2:**

| Criteria | Algorithm | Transition | | |
|---|---|---|---|---|
| | | $0 \rightarrow 1$ | $0 \rightarrow 2$ | $1 \rightarrow 2$ |
| iAUC | msCox | 0.533* ± 0.03 | 0.481* ± 0.03 | 0.489* ± 0.03 |
| | msSplineCox | 0.532* ± 0.03 | 0.479* ± 0.03 | 0.486* ± 0.03 |
| | Linear ex. func. | 0.531* ± 0.03 | 0.510* ± 0.03 | 0.504* ± 0.03 |
| | Example function | **0.580 ± 0.03** | **0.525 ± 0.03** | **0.566 ± 0.03** |
| iBS | msCox | 0.234* ± 0.01 | 0.243* ± 0.01 | **0.159 ± 0.01** |
| | msSplineCox | 0.242* ± 0.01 | 0.251* ± 0.01 | **0.159 ± 0.01** |
| | Linear ex. func. | 0.162* ± 0.01 | 0.161* ± 0.01 | 0.165† ± 0.01 |
| | Example function | **0.146 ± 0.01** | **0.144 ± 0.01** | 0.160 ± 0.01 |

## 7 | APPLICATION ON A REAL CLINICAL DATA SET

The performance of the specific implementation is evaluated based on real illness-death data from one clinical trial in breast cancer.

### 7.1 | Description of the dataset

The performance of the specific implementation is evaluated based on the data from the Molecular Taxonomy of Breast Cancer International Consortium (METABRIC) cohort. For the **METABRIC** dataset, 1903 patients followed for 360 months (30 years) for RFS and OS, with 38% of censoring from state 0 and 13% of censoring from state 1 among patients at risk are included. Descriptive statistics of the dataset are shown in Tables 3 and 4. Table 3 shows descriptive statistics on the number of (No.) observations in the real clinical datasets. Table 4 shows descriptive statistics on the transition times (in months) in the real clinical datasets.

**TABLE 3:**

| Data set | No. observations (%) | | | | | Total |
|---|---|---|---|---|---|---|
| | $0 \rightarrow 1$ | $0 \rightarrow 2$ | $0 \rightarrow$ cens. | $1 \rightarrow 2$ | $1 \rightarrow$ cens. | |
| METABRIC | 677 (36%) | 509 (27%) | 717 (38%) | 593 (88%*) | 84 (12%*) | 1903 |
| * among patients at risk | | | | | | |

**TABLE 4:**

| Data set | Transition | | | | | | | | |
|---|---|---|---|---|---|---|---|---|---|
| | $0 \rightarrow 1$ | | | $0 \rightarrow 2$ | | | $1 \rightarrow 2$ | | |
| | Q1 | median | Q3 | Q1 | median | Q3 | Q1 | median | Q3 |
| METABRIC | 20 | 39 | 81 | 65 | 113 | 121 | 36 | 61 | 110 |

This dataset contains clinical, histo-pathological, gene copy number and gene expression features used to determine breast cancer subgroups. Based on the literature, 17 baseline clinical, histo-pathological and gene copy number features (12 categorical, 5 numerical) are selected. It includes the following features: age, inference on the menopausal status, Nottingham Prognostic Index (NPI), immunohistochemical oestrogen-receptor (ER) status, number of positive lymph nodes, cancer grade, tumor size, tumor histological type, cellularity, Her2 copy number by SNP6, Her2 expression, ER Expression, progesterone (PR) expression, type of breast surgery, cancer molecular subtype (pam50 subgroup, integrative cluster), chemotherapy regimen, hormonal regimen, radiotherapy regimen.

The evaluation may comprise imputing missing values by the median value for numerical features and by the mode for categorical features. The evaluation may comprise applying one-hot encoding on categorical features and standardize numerical features with the Z-score. The evaluation may comprise applying fixing a uniform length for the time intervals to one month such that the time interval for month $j$, $v_j = [j-1, j)$, includes all the events that occurred on the daily time interval $[(j-1)\times30.5, j\times30.5)$. The evaluation may comprise applying fixing $K = 120$ (i.e., $\tau = 120$ months = 10 years) and subdivide the time axis into monthly intervals between 0 and 120 months and set event times after 120 months in a last interval $v_{121}$.

### 7.2 | Gene selection using an independent dataset on breast cancer

Several approaches have been reported to integrate gene expression data into survival models. These approaches are based either on dimension reduction, on gene or metagene selection (see Reference 40) or, more recently, on the use of a large number of gene expression values (> 1000) with the development of deep learning methods (see References 41 and 42). The evaluation may use a selection approach in order to extract a ranked list of cancer-related genes based on their p-values from independent transition-specific Cox P.H. models. The genes are selected using an independent dataset in order to control for selection bias; the evaluation uses the Breast Invasive Carcinoma TCGA PanCancer. The Breast Invasive Carcinoma TCGA PanCancer data set is available at the following URL: https://www.cbioportal.org/study/clinicalData?id=brca_tcga_pan_can_atlas_2 018.

The following two steps describes the selection procedure:

1. For each gene expression feature:

- Fitting independent transition-specific Cox P.H. models including the clinical covariates and each gene expression feature; and
- For each of the three transitions, computing the p-value from a Wald test on the estimated coefficient related to the specific gene.

2. For each transition:

- Adjusting the p-values with a Benjamini-Hochberg multi-test correction for each transition independently;
- Ranking the p-values for each transition; and
- Using different thresholds, noted $\alpha$, of the p-values for gene selection.

The number of gene selected for each threshold is presented in Table 5. Table 5 shows the number of (No.) genes selected per transition on the Breast Invasive Carcinoma TCGA PanCancer dataset for each threshold.

#### TABLE 5:

| Threshold (a) | No. genes selected per transition | | | Total * |
|---|---|---|---|---|
| | $0 \to 1$ | $0 \to 2$ | $1 \to 2$ | |
| 0.001 | 8 | 0 | 1 | 9 |
| 0.005 | 17 | 1 | 1 | 19 |
| 0.01 | 26 | 2 | 1 | 29 |
| 0.05 | 76 | 6 | 3 | 84 |
| 0.1 | 110 | 36 | 11 | 156 |
| * Total number of unique genes selected. | | | | |

#### TABLE 6:

| Term | P-value | A djusted p-value |
|---|---|---|
| **Epithelial Mesenchymal Transition** | **$1.073e^{-9}$** | **$3.325e^{-8}$** |
| Apical Junction | $8.390e^{-4}$ | 0.010 |
| Coagulation | $9.967e^{-4}$ | 0.010 |
| Hypoxia | $4.989e^{-3}$ | 0.039 |
| Hedgehog Signaling | 0.017 | 0.085 |
| Angiogenesis | 0.017 | 0.085 |

(continued)

| Term | P-value | A djusted p-value |
|---|---|---|
| **Epithelial Mesenchymal Transition** | **1.073e$^{-9}$** | **3.325e$^{-8}$** |
| Apical Surface | 0.024 | 0.085 |
| Complement | 0.025 | 0.085 |
| Inflammatory Response | 0.025 | 0.085 |

For the transition $0 \to 1$, several genes are selected (up to 110 genes for the threshold 0.1). While for the transitions $0 \to 2$ and $1 \to 2$, very few genes are selected. In order to assess the biological meaning of the selected genes, the evaluation may comprise performing a gene set enrichment analysis (see Reference 43) for each transition-specific list of genes against the Hallmark (see Reference 44) collection of the Molecular Signatures Database (MSigDB). For the transitions $0 \to 2$ and $1 \to 2$, there is no significant enriched terms. For the transition $0 \to 1$, there are 9 significant enriched terms displayed in Table 6. Table 6 shows significant enriched terms for the list of genes specific to the transition $0 \to 1$. In particular, the most significant enriched term "Epithelial Mesenchymal Transition" is related to a core biological component of the metastatic process in breast cancer (see Reference 45) and therefore well related to a primary cancer progression through a state relapse. Hence, the gene selected on the transition $0 \to 1$ display a coherent functional profile associated to cancer relapse. The addition of the selected genes in an illness-death model is thus expected to improve its performance for the transition $0 \to 1$ more than the others.

### 7.3 | Benchmark

The integrated predictive performances are shown in Tables 7 and 8. The evaluation may comprise comparing different models with different lists of genes varying the threshold $\alpha$ of the p-values. For each of the four algorithms compared, the models show different performances in terms of iAUC. The algorithms msCox and msSplineCox display similar iAUC for the transition $0 \to 1$, better iAUC for the transition $0 \to 2$ and poorer iAUC for the transition $1 \to 2$ when the value of $\alpha$ increases (except for $\alpha = 0.1$). The algorithms of the example function and the Linear example function improve their iAUC for the transition $0 \to 1$ when the value of $\alpha$ increases. As expected, the example function and the Linear example function display similar iAUC for the transitions $0 \to 2$ and $1 \to 2$ when the value of $\alpha$ increases. Hence, the two deep learning methods have a better iAUC for the transition $0 \to 1$ thanks to the addition of the selected gene features in the modelization. This result is consistent with the gene set enrichment analysis realized in Section 7.2.2 in which the list of genes selected for the transition $0 \to 1$ is particularly related to the metastatic process whereas there are no enriched terms in the genes selected for the transitions $0 \to 2$ and $1 \to 2$. Finally, the model $M^{BH}_{0.1}$ is chosen as the best model to predict the transitions $0 \to 1$ and $1 \to 2$. With the model $M^{BH}_{0.1}$, the example function outperforms significantly the other algorithms with an iAUC of 0.714 and an iBS of 0.142 for the transition $0 \to 1$, an iAUC of 0.730 and an iBS of 0.167 for the transition $1 \to 2$. Regarding performance for the transition $0 \to 2$, experiencing transition $0 \to 2$ means that the patient died from another cause than cancer, but the clinical and biological features provided to the model were not related (excluding the age) to non-cancer causes of death.

Table 7 shows integrated AUCs (median $\pm$ sd) on the validation sets (internal validation) for the METABRIC dataset, with a uniform subdivision of the time scale in $K = 120$ (months). The AUC and BS measures are integrated at all the 30 equidistant time points in $[90, \tau]$ (i.e., at every month from 3 months). The model $M_0$ uses only the clinical features and each model $M^{BH}$, for $\alpha \in [0.001\ 0.005, 0.01, 0.05, 0.1]$, uses the clinical features and the genes selected with a corrected p-value less than $\alpha$.

### TABLE 7:

| Criteria | Model | Algorithm | Transition | | |
|---|---|---|---|---|---|
| | | | **0 $\to$ 1** | **0 $\to$ 2** | **1 $\to$ 2** |
| | | | | | |
| | M$_0$ | msCox | 0.702 $\pm$ 0.03 | **0.721* $\pm$ 0.05** | 0.740 $\pm$ 0.04 |
| | | msSplineCox | **0.704 $\pm$ 0.02** | 0.718* $\pm$ 0.05 | **0.744 $\pm$ 0.04** |
| | | Linear ex. func. | 0.654* $\pm$ 0.03 | 0.612* $\pm$ 0.04 | 0.714* $\pm$ 0.05 |
| | | Example function | 0.692 $\pm$ 0.02 | 0.689 $\pm$ 0.04 | 0.737 $\pm$ 0.03 |

(continued)

| Criteria | Model | Algorithm | Transition | | |
|---|---|---|---|---|---|
| | | | 0 → 1 | 0 → 2 | 1 → 2 |
| iAUC | $M_{0.001}^{BH}$ | msCox | $0.700 \pm 0.02$ | $0.730^* \pm 0.05$ | $0.722 \pm 0.03$ |
| | | msSplineCox | $\mathbf{0.701 \pm 0.02}$ | $\mathbf{0.768^* \pm 0.06}$ | $0.728 \pm 0.02$ |
| | | Linear ex. func. | $0.658^* \pm 0.03$ | $0.613^* \pm 0.04$ | $0.706^\ddagger \pm 0.03$ |
| | | Example function | $0.694 \pm 0.03$ | $0.685 \pm 0.04$ | $0.729 \pm \mathbf{0.03}$ |
| | $M_{0.005}^{BH}$ | msCox | $\mathbf{0.703 \pm 0.02}$ | $0.744^* \pm 0.05$ | $0.718 \pm 0.04$ |
| | | msSplineCox | $0.697 \pm 0.02$ | $\mathbf{0.748^\ddagger \pm 0.06}$ | $0.719 \pm 0.04$ |
| | | Linear ex. func. | $0.659^* \pm 0.03$ | $0.635^* \pm 0.04$ | $0.714^\dagger \pm 0.03$ |
| | | Example function | $0.700 \pm 0.03$ | $0.705 \pm 0.03$ | $\mathbf{0.725 \pm 0.04}$ |
| | $M_{0.01}^{BH}$ | msCox | $0.698 \pm 0.02$ | $0.743^* \pm 0.05$ | $0.700^\ddagger \pm 0.04$ |
| | | msSplineCox | $\mathbf{0.706 \pm 0.02}$ | $\mathbf{0.763^* \pm 0.05}$ | $0.704^\dagger \pm 0.04$ |
| | | Linear ex. func. | $0.657^* \pm 0.03$ | $0.640^* \pm 0.04$ | $0.714 \pm 0.03$ |
| | | Example function | $0.698 \pm 0.02$ | $0.695 \pm 0.04$ | $\mathbf{0.728 \pm 0.03}$ |
| | $M_{0.05}^{BH}$ | msCox | $0.702 \pm 0.02$ | $0.736^* \pm 0.05$ | $0.696^* \pm 0.04$ |
| | | msSplineCox | $0.699 \pm 0.02$ | $\mathbf{0.773^\ddagger \pm 0.06}$ | $0.714 \pm 0.04$ |
| | | Linear ex. func. | $0.671^* \pm 0.03$ | $0.639^* \pm 0.03$ | $0.716^\ddagger \pm 0.03$ |
| | | Example function | $\mathbf{0.708 \pm 0.03}$ | $0.687 \pm 0.03$ | $\mathbf{0.732 \pm 0.03}$ |
| | $M_{0.1}^{BH}$ | msCox | $0.677^* \pm 0.03$ | $\mathbf{0.730^* \pm 0.04}$ | $0.669^* \pm 0.04$ |
| | | msSplineCox | $0.685 \pm 0.01$ | $0.683 \pm 0.02$ | $0.688 \pm 0.01$ |
| | | Linear ex. func. | $0.684^* \pm 0.03$ | $0.649^* \pm 0.04$ | $0.720 \pm 0.03$ |
| | | Example function | $\mathbf{0.714 \pm 0.02}$ | $0.690 \pm 0.04$ | $\mathbf{0.730 \pm 0.03}$ |

Table 8 shows integrated BSs (median $\pm$ sd) on the validation sets (internal validation) for the METABRIC dataset, with a uniform subdivision of the time scale in $K$ = 120 (months). The AUC and BS measures are integrated at all the 30 equidistant time points in [90,] (i.e., at every months from 3 months). The model $M_0$ uses only the clinical features and each model $M^{BH}$, for $\alpha \in [0.001, 0.005, 0.01, 0.05, 0.1]$, uses the clinical features and the genes selected with a corrected p-value less than $\alpha$.

**TABLE 8:**

| Criteria | Model | Algorithm | Transition | | |
|---|---|---|---|---|---|
| | | | 0 → 1 | 0 → 2 | 1 → 2 |
| | $M_0$ | msCox | $0.148^\ddagger \pm 0.01$ | $0.066^* \pm 0.01$ | $\mathbf{0.156 \pm 0.01}$ |
| | | msSplineCox | $0.148^\ddagger \pm 0.01$ | $0.068^* \pm 0.01$ | $0.158^* \pm 0.01$ |
| | | Linear ex. func. | $0.150^* \pm 0.01$ | $0.060 \pm 0.01$ | $0.168^\ddagger \pm 0.02$ |
| | | Example function | $\mathbf{0.144 \pm 0.01}$ | $\mathbf{0.058 \pm 0.01}$ | $0.163 \pm 0.01$ |
| | $M_{0.001}^{BH}$ | msCox | $0.145 \pm 0.01$ | $0.064^* \pm 0.01$ | $\mathbf{0.157 \pm 0.01}$ |
| | | msSplineCox | $0.147 \pm 0.01$ | $0.066^* \pm 0.01$ | $0.160 \pm 0.01$ |
| | | Linear ex. func. | $0.146^\dagger \pm 0.01$ | $0.059 \pm 0.01$ | $0.170^\dagger \pm 0.01$ |
| | | Example function | $\mathbf{0.143 \pm 0.01}$ | $\mathbf{0.057 \pm 0.01}$ | $0.162 \pm 0.01$ |
| iBS | $M_{0.005}^{BH}$ | msCox | $0.145 \pm 0.01$ | $0.065^* \pm 0.01$ | $\mathbf{0.160 \pm 0.01}$ |
| | | msSplineCox | $0.149^\dagger \pm 0.01$ | $0.067^* \pm 0.01$ | $0.162 \pm 0.01$ |
| | | Linear ex. func. | $0.145^\dagger \pm 0.01$ | $0.058 \pm 0.01$ | $0.169^\ddagger \pm 0.01$ |
| | | Example function | $\mathbf{0.142 \pm 0.01}$ | $\mathbf{0.056 \pm 0.01}$ | $0.162 \pm 0.01$ |

(continued)

| Criteria | Model | Algorithm | Transition | | |
|---|---|---|---|---|---|
| | | | $0 \rightarrow 1$ | $0 \rightarrow 2$ | $1 \rightarrow 2$ |
| | $M_{0.01}^{BH}$ | msCox | $0.146 \pm 0.01$ | $0.065^* \pm 0.01$ | $\mathbf{0.164 \pm 0.01}$ |
| | | msSplineCox | $0.148 \pm 0.01$ | $0.067^* \pm 0.01$ | $0.166 \pm 0.01$ |
| | | Linear ex. func. | $0.145 \pm 0.01$ | $0.058 \pm 0.01$ | $0.173^\dagger \pm 0.02$ |
| | | Example function | $\mathbf{0.144 \pm 0.01}$ | $\mathbf{0.056 \pm 0.01}$ | $0.165 \pm 0.01$ |
| | $M_{0.05}^{BH}$ | msCox | $0.148^* \pm 0.01$ | $0.067^* \pm 0.01$ | $0.176^* \pm 0.01$ |
| | | msSplineCox | $0.153^\ddagger \pm 0.01$ | $0.069^* \pm 0.00$ | $0.175^\ddagger \pm 0.01$ |
| | | Linear ex. func. | $0.144^\ddagger \pm 0.01$ | $\mathbf{0.058 \pm 0.01}$ | $0.176^* \pm 0.02$ |
| | | Example function | $\mathbf{0.142 \pm 0.01}$ | $\mathbf{0.058 \pm 0.01}$ | $\mathbf{0.163 \pm 0.01}$ |
| | $M_{0.1}^{BH}$ | msCox | $0.158^* \pm 0.01$ | $0.069^* \pm 0.01$ | $0.202^* \pm 0.01$ |
| | | msSplineCox | $0.162^\dagger \pm 0.01$ | $0.066 \pm 0.00$ | $0.201^\dagger \pm 0.01$ |
| | | Linear ex. func. | $\mathbf{0.142 \pm 0.01}$ | $0.058 \pm 0.01$ | $0.172^\ddagger \pm 0.02$ |
| | | Example function | $\mathbf{0.142 \pm 0.01}$ | $\mathbf{0.057 \pm 0.01}$ | $\mathbf{0.164 \pm 0.01}$ |

## 8 | ANALYSIS OF RESULTS

The example function forms a novel deep learning method to model an illness-death process and to predict two-stages evolution of a disease based on baseline covariates. It is the first deep learning architecture developed in the context of multi-state analysis. It outperforms standard methodologies in this context. In clinical practice, the example function may be useful in personalized medicine by providing predictions of the risks of relapse and death. It could help physicians to adapt the therapeutic guidelines for a specific patient.

Prognostication of diseases is a key momentum in the medical decision process of various diseases, for example to identify populations at risk of cardiovascular complications or to identify populations at risk of cancer relapse. The most commonly used approaches are based on nomograms and scores computed based on a few clinical and biological features. For example, the CHAD2-DS2VASC score is commonly used by physicians to identify patients who require anticoagulant treatment following the diagnosis of a cardiac atrial arrhythmia (Reference 48). In the same way, the RSClin tool that combines clinical, pathological and genetic information is commonly used in oncology to predict the risk of breast cancer relapse and to determine more precisely which patients need chemotherapy in addition to surgery (see Reference 49). But a lot of medical information contained in the patients' records is left unexploited. As opposed to the development of new biomarkers relying on expensive and highly specialized technologies, another approach is to focus on the optimization of prognostic models based on large but accessible information. In the area of digital medicine which aims to capture and combine a huge amount of data, the specialized algorithm of the example function supports practices of healthcare workers based on standardized guidelines on the one hand, and on personalized assessment on the other hand.

The example function uses a multi-task architecture and transition-specific subnetworks to learn an estimation of the density probabilities of occurrence of state transitions of an illness death process. It uses piecewise approximations to provide accurate predictions of the cumulative probabilities of state transitions. The example function uses multiple fully connected layers and non-linear activation functions to model the relationships between covariates and risks of transitions without any assumption. It is trained by minimizing a loss function designed to both capture the relationships between covariates and risks of transitions and provide smooth piecewise approximations of the density probabilities.

Through experiments on simulated datasets, the investigation of different configurations of the example function illustrates the benefit of the loss function. A comparison of the predictive performances of the method with the state-of-the-art methods using discrimination and calibration criteria and an evaluation of the example function in predicting the cumulative probabilities of state transitions on a simulated dataset and on real datasets on breast cancer show that the example function provides significant improvements in comparison with the other methods when non-linear patterns are found in the data. It has to be noted that the results may even be improved with more data, as the datasets used in the tests were relatively small, and the number of medical characteristics relatively low.

Furthermore, the combination of heterogeneous individual features improves medical decision making. On the real dataset on breast cancer, section 7 illustrates how the example function can be easily adapted to integrate various types of data (as clinical, biological, molecular, gene expression) and displays in this case coherent and significant improvements in comparison with statistical methods.

Time-to-event data collected in a clinical setting can suffer from a high censoring rate, as in the case of rare diseases. Applying survival models on datasets with fewer events than censored observations can make difficult the modeling of the relation between covariates and risks of events. In addition, the use of a larger amount of training data improves deep learning methods. Thus, developing a reliable multi-state model with few events and a small training is challenging and can results in a poor predictive model. This is often the case with datasets from clinical trials where the number of patients is relatively low compared to the databases commonly used in deep learning. The architecture of the example function is adapted to such datasets from clinical trials because of the use of the hyper-parameters to simplify the architecture according to the training data available for each of the three transitions. Moreover, given the increasing popularity of using real-world data (RWD) collection, such as electronic health records (EHRs) or disease registries, the specific implementation aims to exploit these data in the future for the problematic to make more efficient and reliable decision-making.

The example function has been developed for the application on an illness-death process and may be applied in many cancers to predict two-stages evolution. The method may be generalized for more complex disease processes by adapting the states and transitions.

**9 |SYSTEM**

Any method herein is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

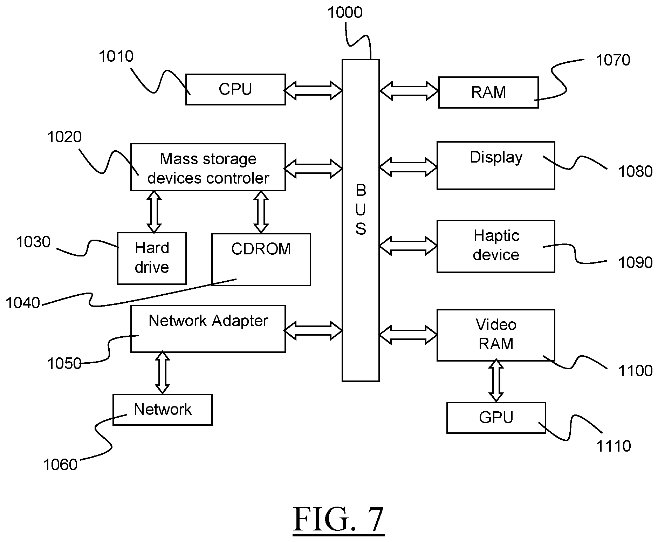

FIG. 7 shows an example of the system, wherein the system is a client computer system, e.g., a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks; and CD-ROM disks 1040. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method.

**Claims**

1. A computer-implemented method for machine-learning a function configured, based on input covariates representing medical characteristics of a patient, with respect to a multi-state model of an illness having states and transitions between the states, to output a distribution of transition-specific probabilities for each interval of a set of intervals, the set of intervals forming a subdivision of a follow-up period, the distribution of transition-specific probabilities being piecewise constant,

   wherein the multi-state model is an illness-death model,
   wherein the illness is a cancer disease, or a disease **characterized by** an intermediate state and a final state,
   wherein the medical characteristics comprise characteristics representing a general state of the patient and/or characteristics representing a condition of the patient with respect to the illness,
   wherein the function comprises a covariate-shared subnetwork and a transition-specific subnetwork per transition,
   wherein the covariate-shared subnetwork comprises a respective fully connected neural network and each transition-specific subnetwork comprises a fully connected neural network,
   wherein the covariate-shared subnetwork comprises a respective non-linear activation function and each transition-specific subnetwork comprises a respective non-linear activation function,
   wherein each transition-specific subnetwork is followed by a softmax layer, wherein the multi-state model comprises competing transitions, and the transition-specific subnetworks of the competing transitions share a common softmax layer,
   the machine-learning method comprising:

   - providing an input dataset of covariates and time-to-event data of a set of patients; and
   - training the function based on the input dataset, wherein the training comprises minimizing a loss function which includes:

     ▪ a likelihood term, and
     ▪ a regularization term that penalizes:

       o in weight matrices, first order differences of weights associated with two adjacent time intervals, and/or
       o in bias vectors, first order differences of biases associated with two adjacent time intervals.

2. The machine-learning method of claim 1, wherein the illness is a breast cancer.

3. A method of use of a function machine-learnt according to the method of claim 1 or 2, the use method comprising:

   - providing input covariates representing medical characteristics of a patient; and
   - applying the function to the input covariates, thereby outputting, with respect to a multi-state model of an illness having states and transitions between the states, a distribution of transition-specific probabilities for each interval of a set of time intervals, the set of time intervals forming a subdivision of a follow-up period.

4. The use method of claim 3, further comprising, based on the outputted distribution of transition-specific probabilities:

   - computing one or more transition-specific cumulative incidence functions (CIF), and optionally displaying said one or more transition-specific cumulative incidence functions;
   - identifying relapse risk and/or death risk associate to the patient; and/or
   - determining a treatment, a treatment adaptation, a follow-up visit, and/or a surveillance with diagnostic tests, for example based on the identified relapse risk and/or death risk.

5. A computer program including instructions for performing the method of claim 1 or 2, and/or the method of claim 3 or 4.

6. A device comprising a data storage medium having recorded thereon the computer program of claim 5.

7. The device of claim 6, further comprising a processor coupled to the data storage medium.

**Patentansprüche**

1. Computerimplementiertes Verfahren zum maschinellen Lernen einer Funktion, die konfiguriert ist, um basierend auf Eingangs-Kovariaten, die medizinische Merkmale eines Patienten darstellen, in Bezug auf ein Mehrzustandsmodell einer Krankheit, das Zustände und Übergänge zwischen den Zuständen aufweist, eine Verteilung von übergangsspezifischen Wahrscheinlichkeiten für jedes Intervall einer Menge von Intervallen auszugeben, wobei der Satz von Intervallen eine Unterteilung einer Nachbeobachtungsperiode bildet, wobei die Verteilung von übergangsspezifischen Wahrscheinlichkeiten stückweise konstant ist,

   wobei das Mehrzustandsmodell ein Krankheit-Tod-Modell ist,
   wobei die Krankheit eine Krebserkrankung oder eine Erkrankung ist, die durch einen Zwischenzustand und einen Endzustand gekennzeichnet ist,
   wobei die medizinischen Merkmale Merkmale umfassen, die einen allgemeinen Zustand des Patienten darstellen und/oder Merkmale, die einen Zustand des Patienten in Bezug auf die Krankheit darstellen,
   wobei die Funktion ein gemeinsames Kovariaten-Teilnetz und ein übergangsspezifisches Teilnetz pro Übergang umfasst,
   wobei das kovariatengeteilte Teilnetz ein entsprechendes vollständig verbundenes neuronales Netz umfasst und jedes übergangsspezifische Teilnetz ein vollständig verbundenes neuronales Netz umfasst,
   wobei das kovariatengeteilte Teilnetz eine jeweilige nichtlineare Aktivierungsfunktion umfasst und jedes übergangsspezifische Teilnetz eine jeweilige nichtlineare Aktivierungsfunktion umfasst,
   wobei auf jedes übergangsspezifische Teilnetz eine Softmax-Schicht folgt, wobei das Mehrzustandsmodell konkurrierende Übergänge umfasst und die übergangsspezifischen Teilnetze der konkurrierenden Übergänge eine gemeinsame Softmax-Schicht teilen,
   das Verfahren maschinellen Lernens umfassend:

   - Bereitstellen eines Eingangsdatensatzes von Kovariaten und Zeit-zu-Ereignis-Daten einer Gruppe von Patienten; und
   - Trainieren der Funktion basierend auf dem Eingabedatensatz, wobei das Trainieren ein Minimieren einer Verlustfunktion umfasst, die Folgendes beinhaltet:

     ▪ einen Wahrscheinlichkeitsterm und
     ▪ einen Regularisierungsterm, der Folgendes bestraft:

       ∘ in Gewichtsmatrizen, Differenzen erster Ordnung von Gewichten, die mit zwei benachbarten Zeitintervallen assoziiert sind, und/oder
       ∘ in Bias-Vektoren, Differenzen erster Ordnung von Bias, die mit zwei benachbarten Zeitintervallen assoziiert sind.

2. Maschinelles Lernverfahren nach Anspruch 1, wobei die Krankheit Brustkrebs ist.

3. Verwendungsverfahren einer maschinell gelernten Funktion gemäß dem Verfahren nach Anspruch 1 oder 2, das Verwendungsverfahren umfassend:

   - Bereitstellen von Eingangs-Kovariaten, die medizinische Merkmale eines Patienten darstellen; und
   - Anwenden der Funktion auf die Eingangs-Kovariaten, wodurch in Bezug auf ein Mehrzustandsmodell einer Krankheit, das Zustände und Übergänge zwischen den Zuständen aufweist, eine Verteilung von übergangsspezifischen Wahrscheinlichkeiten für jedes Intervall eines Satzes von Zeitintervallen ausgegeben wird, wobei der Satz von Zeitintervallen eine Unterteilung einer Nachbeobachtungsperiode bildet.

4. Verwendungsverfahren nach Anspruch 3, ferner umfassend, basierend auf der ausgegebenen Verteilung der übergangsspezifischen Wahrscheinlichkeiten:

   - Berechnen einer oder mehrerer übergangsspezifischer kumulativer Inzidenzfunktionen (CIF) und optional Anzeigen der einen oder mehreren übergangsspezifischen kumulativen Inzidenzfunktionen;
   - Identifizieren eines Rückfallrisikos und/oder eines mit dem Patienten assoziierten Todesrisikos; und/oder
   - Bestimmen einer Behandlung, einer Behandlungsanpassung, einer Nachuntersuchung und/oder einer Überwachung mit diagnostischen Tests, zum Beispiel basierend auf dem identifizierten Rückfallrisiko und/oder Sterberisiko.

**5.** Computerprogramm, das Anweisungen zum Durchführen des Verfahrens nach Anspruch 1 oder 2 und/oder des Verfahrens nach Anspruch 3 oder 4 beinhaltet.

**6.** Vorrichtung, umfassend ein Datenspeichermedium, auf dem das Computerprogramm nach Anspruch 5 aufgezeichnet ist.

**7.** Vorrichtung nach Anspruch 6, ferner umfassend einen Prozessor, der mit dem Datenspeichermedium gekoppelt ist.

**Revendications**

**1.** Procédé mis en œuvre par ordinateur pour l'apprentissage automatique d'une fonction configurée, sur la base de covariables d'entrée représentant des caractéristiques médicales d'un patient, par rapport à un modèle multi-états d'une maladie présentant des états et des transitions entre les états, pour produire une distribution de probabilités spécifiques aux transitions pour chaque intervalle d'un ensemble d'intervalles, l'ensemble d'intervalles formant une subdivision d'une période de suivi, la distribution de probabilités spécifiques aux transitions étant constante par morceaux,

dans lequel le modèle multi-états est un modèle maladie-décès,
dans lequel la maladie est un cancer, ou une maladie **caractérisée par** un état intermédiaire et un état final,
dans lequel les caractéristiques médicales comprennent des caractéristiques représentant un état général du patient et/ou des caractéristiques représentant un état du patient par rapport à la maladie,
dans lequel la fonction comprend un sous-réseau partagé par covariable et un sous-réseau spécifique à une transition par transition,
dans lequel le sous-réseau partagé par covariable comprend un réseau neuronal respectif entièrement connecté et chaque sous-réseau spécifique à une transition comprend un réseau neuronal entièrement connecté,
dans lequel le sous-réseau partagé par covariable comprend une fonction d'activation non linéaire respective et chaque sous-réseau spécifique à une transition comprend une fonction d'activation non linéaire respective,
dans lequel chaque sous-réseau spécifique à une transition est suivi d'une couche softmax,
dans lequel le modèle multi-états comprend des transitions concurrentes, et les sous-réseaux spécifiques aux transitions des transitions concurrentes partagent une couche softmax commune,
le procédé d'apprentissage automatique comprenant :

- la fourniture d'un ensemble de données d'entrée de covariables et de données de temps jusqu'à événement d'un ensemble de patients ; et
- l'apprentissage de la fonction sur la base de l'ensemble de données, dans lequel l'apprentissage comprend la minimisation d'une fonction de perte qui inclut :

• un terme de vraisemblance, et
• un terme de régularisation qui pénalise :

o dans des matrices de pondération, des différences de premier ordre de pondérations associées à deux intervalles de temps adjacents, et/ou
o dans des vecteurs de biais, des différences de premier ordre de biais associées à deux intervalles de temps adjacents.

**2.** Procédé d'apprentissage automatique selon la revendication 1, dans lequel la maladie est un cancer du sein.

**3.** Procédé d'utilisation d'une fonction apprise automatiquement selon le procédé de la revendication 1 ou 2, le procédé d'utilisation comprenant :

- la fourniture de covariables d'entrée représentant des caractéristiques médicales d'un patient ; et
- l'application de la fonction aux covariables d'entrée, produisant ainsi, par rapport à un modèle multi-états d'une maladie présentant des états et des transitions entre les états, une distribution de probabilités spécifiques aux transitions pour chaque intervalle d'un ensemble d'intervalles de temps, l'ensemble d'intervalles de temps formant une subdivision d'une période de suivi.

**4.** Procédé d'utilisation selon la revendication 3, comprenant en outre, sur la base de la distribution produite de probabilités spécifiques aux transitions :

- le calcul d'une ou plusieurs fonctions d'incidence cumulée (FIC) spécifiques aux transitions, et optionnellement l'affichage desdites une ou plusieurs fonctions d'incidence cumulée spécifiques aux transitions ;
- l'identification du risque de récidive et/ou du risque de décès associé au patient ; et/ou
- la détermination d'un traitement, d'une adaptation de traitement, d'une visite de suivi et/ou d'une surveillance avec des tests de diagnostic, par exemple sur la base du risque de récidive et/ou du risque de décès identifié(s).

5. Programme informatique incluant des instructions pour réaliser le procédé selon la revendication 1 ou 2 et/ou le procédé selon la revendication 3 ou 4.

6. Dispositif comprenant un support de stockage de données sur lequel est enregistré le programme informatique selon la revendication 5.

7. Dispositif selon la revendication 6, comprenant en outre un processeur couplé au support de stockage de données.

$f_{01}(t)$

State0
Event-free

State1
Relapse

State2
Death

$f_{02}(t)$

$f_{12}(t - T_0 \mid T_0, D_0 = 1)$

## FIG. 1

$a_0 = 0$     $a_1$     $a_2$               $a_{K-1}$     $a_K = \tau$     Time $(t)$

## FIG. 2

## FIG. 3

FIG. 4

FIG. 5

(a) $\hat{F}_{01}(.| X)$ estimated with the example function.

(b) $\hat{F}_{02}(.| X)$ estimated with the example function.

(b) $\hat{F}_{12}(.| X)$ estimated with the example function.

(d) $\hat{F}_{01}(.| X)$ estimated with the example function ($P_\lambda = 0$).

(e) $\hat{F}_{02}(.| X)$ estimated with the example function ($P_\lambda = 0$).

(f) $\hat{F}_{12}(.| X)$ estimated with the example function ($P_\lambda = 0$).

## FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WEBSTER AJ.** Multi-stage models for the failure of complex systems, cascading disasters, and the onset of disease.. *PloS one*, 2019, vol. 14 (5), e0216422
- **EULENBURG C** ; **MAHNER S** ; **WOELBER L** ; **WEGSCHEIDER K.** A systematic model specification procedure for an illness-death model without recovery.. *PloS one*, 2015, vol. 10 (4), e0123489
- **IACOBELLI S** ; **CARSTENSEN B.** Multiple time scales in multi-state models. *Statistics in medicine*, 2013, vol. 32 (30), 5315-5327
- **COMMENGES D** ; **JOLY P** ; **LETENNEUR L** ; **DARTIGUES JF.** Incidence and mortality of Alzheimer's disease or dementia using an illness-death model. *Statistics in medicine*, 2004, vol. 23 (2), 199-210
- **RAMEZANKHANI A** ; **BLAHA MJ** ; **MIRBOLOUK HM** ; **AZIZI F** ; **HADAEGH F.** Multi-state analysis of hypertension and mortality: application of semi-Markov model in a longitudinal cohort study.. *BMC Cardiovascular Disorders*, 2020, vol. 20 (1), 1-13
- **KAPLAN EL** ; **MEIER P.** Nonparametric estimation from incomplete observations.. *Journal of the American statistical association*, 1958, vol. 53 (282), 457-481
- **AALEN O.** Nonparametric inference for a family of counting processes.. *The Annals of Statistics*, 1978, 701-726
- **COX DR.** Regression models and life-tables.. *Journal of the Royal Statistical Society: Series B (Methodological)*, 1972, vol. 34 (2), 187-202
- **DE WREEDE LC** ; **FIOCCO M** ; **PUTTER H.** The mstate package for estimation and prediction in non- and semi-parametric multistate and competing risks models.. *Computer methods and programs in biomedicine*, 2010, vol. 99 (3), 261-274
- **ANDERSEN PK** ; **BORGAN O** ; **GILL RD** ; **KEIDING N.** Statistical models based on counting processes.. *Springer Science & Business Media .*, 2012
- **FARAGGI D** ; **SIMON R.** A neural network model for survival data.. *Statistics in medicine*, 1995, vol. 14 (1), 73-82
- **LUCK M** ; **SYLVAIN T** ; **CARDINAL H** ; **LODI A** ; **BENGIO Y.** Deep learning for patient-specific kidney graft survival analysis.. *arXiv preprint arXiv:1705.10245*, 2017

- **KATZMAN JL** ; **SHAHAM U** ; **CLONINGER A** ; **BATES J** ; **JIANG T** ; **KLUGER Y.** DeepSurv: personalized treatment recommender system using a Cox proportional hazards deep neural network.. *BMC medical research methodology*, 2018, vol. 18 (1), 24
- **FOTSO S.** Deep neural networks for survival analysis based on a multi-task framework.. *arXiv preprint arXiv:1801.05512*, 2018
- **KVAMME H** ; **BORGAN Ø** ; **SCHEEL I.** Time-to-event prediction with neural networks and Cox regression.. *Journal of Machine Learning Research*, 2019, vol. 20 (129), 1-30
- **GIUNCHIGLIA E** ; **NEMCHENKO A** ; **SCHAAR V. DM.** Rnn-surv: A deep recurrent model for survival analysis. Springer, 2018, 23-32
- **REN, K** ; **QIN, J.** ; **ZHENG, L.** ; **YANG, Z.** ; **ZHANG, W** ; **QIU, L.** ; **YU, Y.** Deep Recurrent Survival Analysis.. *Proceedings of the AAAI Conference on Artificial Intelligence*, 2019, vol. 33 (01), 4798-4805
- **GENSHEIMER MF** ; **NARASIMHAN B.** A scalable discrete-time survival model for neural networks.. *PeerJ*, 2019, vol. 7, e6257
- **LEE C** ; **ZAME WR** ; **YOON J** ; **SCHAAR V. DM.** *Deephit: A deep learning approach to survival analysis with competing risks.*, 2018
- **KALBFLEISCH JD** ; **PRENTICE RL.** The statistical analysis of failure time data.. John Wiley & Sons, 2011, vol. 360
- **FRIEDMAN M.** Piecewise exponential models for survival data with covariates.. *The Annals of Statistics*, 1982, vol. 10 (1), 101-113
- **KVAMME H** ; **BORGAN Ø.** Continuous and discrete-time survival prediction with neural networks.. *arXiv preprint arXiv:1910.06724*, 2019
- **ANDERSEN PK** ; **BORGAN Ø.** Counting Process Models for Life History Data: A Review.. *Preprint series. Statistical Research Report*, 1984, http://urn.nb. no/URN: NBN: no-23420
- **MEIRA-MACHADO L** ; **UÑA-ÁLVAREZ DJ** ; **CADARSO-SUÁREZ C** ; **ANDERSEN PK.** Multi-state models for the analysis of time-to-event data.. *Statistical methods in medical research*, 2009, vol. 18 (2), 195-222
- **MEIRA-MACHADO L** ; **SESTELO M.** Estimation in the progressive illness-death model: A nonexhaustive review. *Biometrical Journal*, 2019, vol. 61 (2), 245-263

- **TRIEBEL H.** Theory of Function Spaces. *Monographs in mathematics*, 1983, vol. 78
- **PUTTER H** ; **FIOCCO M** ; **GESKUS RB.** Tutorial in biostatistics: competing risks and multi-state models.. *Statistics in medicine*, 2007, vol. 26 (11), 2389-2430
- **RUDER S.** An overview of multi-task learning in deep neural networks.. *arXiv preprint arXiv:1706.05098*, 2017
- **LEE C** ; **YOON J** ; **VAN DER SCHAAR M.** Dynamic-deephit: A deep learning approach for dynamic survival analysis with competing risks based on longitudinal data.. *IEEE Transactions on Biomedical Engineering*, 2019, vol. 67 (1), 122-133
- **MÖST S.** Regularization in discrete survival models.. *PhD thesis. lmu*, 2014
- **TIBSHIRANI R** ; **SAUNDERS M** ; **ROSSET S** ; **ZHU J** ; **KNIGHT K.** Sparsity and smoothness via the fused lasso.. *Journal of the Royal Statistical Society: Series B (Statistical Methodology)*, 2005, vol. 67 (1), 91-108
- **AALENOO** ; **JOHANSENS.** An empirical transition matrix for non-homogeneous Markov chains based on censored observations.. *Scandinavian Journal of Statistics*, 1978, 141-150
- **JACQMIN-GADDA H** ; **BLANCHE P** ; **CHARY E** ; **TOURAINE C** ; **DARTIGUES JF.** Receiver operatingcharacteristic curveestimation for time to event with semicompeting risks and interval censoring.. *Statistical methods in medical research*, 2016, vol. 25 (6), 2750-2766
- **SPITONI C** ; **LAMMENS V** ; **PUTTER H.** Prediction errors for state occupation and transition probabilities in multi-state models.. *Biometrical Journal*, 2018, vol. 60 (1), 34-48
- **JACKSON CH**. flexsurv: a platform for parametric survival modeling in R. *Journal of statistical software*, 2016, vol. 70
- **ROYSTON P** ; **ALTMAN DG.** External validation of a Cox prognostic model: principles and methods.. *BMC medical research methodology*, 2013, vol. 13 (1), 33
- **BERGSTRA J** ; **BENGIO Y.** Random search for hyper-parameter optimization.. *The Journal of Machine Learning Research*, 2012, vol. 13 (1), 281-305
- **WILCOXON F.** Individual comparisons by ranking methods.. Springer, 1992, 196-202
- **BENDER R** ; **AUGUSTIN T** ; **BLETTNER M.** Generating survival times to simulate Cox proportional hazards models.. *Statistics in medicine*, 2005, vol. 24 (11), 1713-1723
- **VAN WIERINGEN WN** ; **KUN D** ; **HAMPEL R** ; **BOULESTEIX AL.** Survival prediction using gene expression data: a review and comparison.. *Computational statistics & data analysis*, 2009, vol. 53 (5), 1590-1603
- **YOUSEFI S** ; **AMROLLAHI F** ; **AMGAD M et al.** Predicting clinical outcomes from large scale cancer genomic profiles with deep survival models.. *Scientific reports*, 2017, vol. 7 (1), 1-11
- **CHING T** ; **ZHU X** ; **GARMIRE LX.** Cox-nnet: an artificial neural network method for prognosis prediction of high-throughput omics data.. *PLoS computational biology*, 2018, vol. 14 (4), e1006076
- **KULESHOV MV** ; **JONES MR** ; **ROUILLARD AD et al.** Enrichr: a comprehensive gene set enrichment analysis web server 2016 update.. *Nucleic acids research*, 2016, vol. 44 (W1), W90-W97
- **LIBERZON A** ; **BIRGER C** ; **THORVALDSDÓTTIR H** ; **GHANDI M** ; **MESIROV JP** ; **TAMAYO P.** The molecular signatures database hallmark gene set collection.. *Cell systems*, 2015, vol. 1 (6), 417-425
- **WU Y** ; **SARKISSYAN M** ; **VADGAMA JV.** Epithelial-mesenchymal transition and breast cancer. *Journal of clinical medicine*, 2016, vol. 5 (2), 13
- **LEE DS** ; **EZEKOWITZ JA.** Risk stratification in acute heart failure.. *Canadian Journal of Cardiology*, 2014, vol. 30 (3), 312-319
- **PARK S** ; **HAN W** ; **KIM J et al.** Risk factors associated with distant metastasis and survival outcomes in breast cancer patients with locoregional recurrence.. *Journal of breast cancer*, 2015, vol. 18 (2), 160
- **LIP GY** ; **NIEUWLAAT R** ; **PISTERS R** ; **LANE DA** ; **CRIJNS HJ.** Refining clinical risk stratification for predicting stroke and thromboembolism in atrial fibrillation using a novel risk factor-based approach: the euro heart survey on atrial fibrillation.. *Chest*, 2010, vol. 137 (2), 263-272
- **SPARANO JA** ; **CRAGER MR** ; **TANG G** ; **GRAY RJ** ; **STEMMER SM** ; **SHAK S**. Development and validation of a tool integrating the 21gene recurrence score and clinical-pathological features to individualize prognosis and prediction of chemotherapy benefit in early breast cancer.. *Journal of Clinical Oncology*, 2020, JCO-20
- **ANCONA M** ; **CEOLINI E** ; **ÖZTIRELI C** ; **GROSS M.** Towards better understanding of gradient-based attribution methods for deep neural networks. *arXiv preprint arXiv:1711.06104*, 2017
- **JACKSON C.** *Multi-state modeling with R: the msm package.*, 2007, 1-53
- **GROHA, S.** ; **SCHMON, S.M.** ; **GUSEV, A.** A general framework for survival analysis and multi-state modelling.. *arXiv preprint arXiv:2006.04893*, 2020